# EUROPEAN PATENT APPLICATION

(11) **EP 1 634 962 A1**
(43) Date of publication of application: **15.03.2006**
(21) Application number: 04722056.1
(22) Date of filing: 19.03.2004
(51) Int. Cl.: C12Q 1/68, C12N 15/09

(54) **METHOD OF DETECTING TARGET NUCLEOTIDE SEQUENCE, DETECTION TARGET STRUCTURE TO BE USED IN EMBODYING THE METHOD, PROCESS FOR PRODUCING THE SAME AND ASSAY KIT FOR DETECTING TARGET NUCLEOTIDE SEQUENCE**

(30) Priority: 26.03.2003 JP 2003085670
(71) Applicant: KABUSHIKI KAISHA TOSHIBA, Tokyo 105-8001 (JP)
(72) Inventor: HORIUCHI, Hideki, c/o Toshiba Corporation, Tokyo 105-8001 (JP); HASHIMOTO, Koji, c/o Toshiba Corporation, Tokyo 105-8001 (JP)
(74) Representative: Granleese, Rhian Jane
(86) International application number: PCT/JP2004/003777
(87) International publication number: WO 2004/085680

(57) **Abstract**

The present invention provides a method of preparing a detection target structure useful for detecting a target nucleotide sequence. For example, provided is a method of preparing a detection target structure from a target nucleic acid (1). The target nucleic acid (1) includes a target nucleotide sequence 2 in a part (FIG. 1A). According to the method of the present invention, a nucleic acid is elongated by using a primer so as to permit the target nucleic acid (1) to have a double strand structure in the portion other than the target nucleotide sequence 2, thereby affording a detection target structure 4 (FIG. 1B). The present invention also provides the detection target structure thus prepared a detection method of the target nucleotide sequence by using the detection target structure, a kit for preparing the detection target structure, and an assay kit for detecting the target nucleotide sequence.

## Description

### Technical Field

The present invention relates to a method of preparing a detection target structure useful for detecting the target nucleotide sequence, the detection target structure prepared by the particular preparing method, a method of detecting the target nucleotide sequence by using the detection target structure, a kit for preparing the detection target structure, and an assay kit for detecting the target nucleotide sequence.

### Background Art

The method generally employed for detecting a particular nucleic acid strand comprising a specified nucleotide sequence includes, for example, a method in which the nucleic acid strand to be detected is amplified by using a pair of primers complementary to two different regions of the nucleic acid strand, followed by applying an electrophoresis so as to detect the presence of the amplified nucleic acid strand, and another method which performs hybridization reaction between the target nucleotide sequence to be detected and a nucleic acid probe that is complementary to the target nucleotide sequence to be detected and immobilized on a solid support. Also, in general, the primer and the nucleic acid probe used for the particular detection are oligonucleotides having a specific consecutive sequence of at least 15 bases. However, even if such the particular oligonucleotides can be designed by utilizing the known primer or the known nucleic acid probe design software, it is possible for a higher order structure to be present in the target nucleotide sequence or for a partially resembling sequence to be present in the reaction system. In such a case, a miss-annealing or a self-hybridization tends to be brought about. In such a conventional method, it is difficult to detect the target nucleotide sequence efficiently with a high accuracy. Also, time-consuming and expense works are required for setting the optimum detection conditions.

The method for detecting the target nucleotide sequence efficiently with a high sensitivity includes, for example, there has been proposed "A hybridization method" disclosed in Japanese Patent Disclosure (Kokai) No. 6-70799 and "DNA detection method utilizing a partial double strand DNA", which is disclosed in Japanese Patent Disclosure No. 11-332566. In these methods, a nucleic acid probe having a sequence complementary to a partial region of the target nucleic acid to be detected is hybridized with the target nucleic acid. What should be noted is that, upon the hybridization reaction, addition is made of another protective nucleic acid strand complementary to the sequence of any portion of the target nucleic acid other than the region which hybridizes with the nucleic acid probe before or during the hybridization reaction, followed by performing the annealing so as to form a double strand in a part. Each of these methods is intended to prevent a non-specific hybridization by allowing the target nucleic acid to be arranged such that the region recognized by the nucleic acid probe is of a single stranded structure and the other region is of a double stranded structure.

However, many problems must be solved before the methods described above are put into the practical use, as pointed out below.
(1) Since it is absolutely necessary to prepare two kinds of molecules of the target nucleic acid and the protective strand under the state of a single strand, the number of process steps required is increased.
(2) The design of the target nucleotide sequence is limited. In order to avoid the non-specific hybridization, it is desirable for any free single strand nucleic acid molecule other than the nucleic acid probe to be present in as little quantity as possible in the hybridization reaction liquid. However, the protective strands are used to be subjected to the annealing. Then, when the target nucleic acid and the protective strand are annealed, these long nucleic acid molecules within the reaction liquid are coupled depending on molecular motions thereof. As a result, ideal annealing may fail under the influences such as the intramolecular higher order structure, which in turn markedly lowers the efficiency of the hybridization. For preventing the difficulty, the design of the target nucleotide sequence is limited.
(3) A long time is required for preparing the target nucleic acid. It should be noted that the process for carrying out the annealing reaction over a long time is absolutely necessary in order to form an accurate base pair between the protective strand and the target nucleic acid, with the result that a long time is required for preparing the target nucleic acid structure as pointed out above.
(4) It is necessary to prepare a target strand and a protective strand by using an asymmetric PCR in, particularly, "DNA detection method utilizing the partial double strand DNA", which is disclosed in Japanese Patent Disclosure No. 11-332566 referred to previously. It follows that, even if a qualitative analysis that permits overcoming the problem pointed out in item (2) above, has been achieved, it is impossible to arrive at the quantitative analysis having high social needs.

### Disclosure of Invention

An object of the present invention is to provide a method of preparing a detection target structure useful for detecting the target nucleotide sequence, a detection target structure prepared by the particular preparing method, a method of detecting the target nucleotide sequence using the particular detection target structure, and an assay kit for detecting the target nucleotide sequence.

The object described above can be achieved in the present invention by the means summarized in the following.

According to a first aspect of the present invention, there is provided a method of preparing a detection target structure useful for detecting the target nucleotide sequence, the method comprising:
reacting a target nucleic acid which contains a target nucleotide sequence positioned in the vicinity of 3'-terminal thereof, under the conditions that permit an appropriate elongation reaction, with;
a primer complementary to the sequence of a region of the target nucleic acid, the region being positioned on the 5'-terminal side relative to the 5'-terminal of the target nucleotide sequence, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to a second aspect of the present invention, there is provided a method of preparing a detection target structure useful for detecting the target nucleotide sequence, comprising:
reacting a target nucleic acid which contains a target nucleotide sequence positioned in the vicinity of 5'-terminal thereof, under the conditions that permit an appropriate elongation reaction, with;
a stopper nucleic acid complementary to the sequence of a region of the target nucleic acid, the region being positioned on the 3'-terminal side relative to the 3'-terminal of the target nucleotide sequence, and the stopper nucleic acid including a modification incapable of elongation at the 3'-terminal thereof,
a primer complementary to the sequence in the vicinity of the 3'-terminal of the target nucleic acid, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to a third aspect of the present invention, there is provided a method of preparing a detection target structure useful for detecting the target nucleotide sequence, comprising:
reacting a first target nucleic acid which contains a first target nucleotide sequence positioned in the vicinity of 3'-terminal thereof, and a second target nucleic acid which is complementary to the first target nucleic acid and contains a second target nucleotide sequence positioned in the vicinity of 5'-terminal thereof, under the conditions that permit obtaining an appropriate elongation reaction, with;
a first primer complementary to the sequence of a region of the first target nucleic acid, the region being positioned on the 5'-terminal side relative to the 5'-terminal of the first target nucleotide sequence,
a stopper nucleic acid complementary to the sequence of a region of the second target nucleic acid, the region being positioned on the 3'-terminal side relative to the 3'-terminal of the second target nucleotide sequence, and the stopper nucleic acid including a modification incapable of elongation at the 3'-terminal thereof,
a second primer complementary to the sequence in the vicinity of the 3'-terminal of the second target nucleic acid, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to a fourth aspect of the present invention, there is provided a method of preparing a detection target structure useful for detecting the target nucleotide sequence, comprising:
reacting a target nucleic acid which contains a first target nucleotide sequence positioned in the vicinity of 3'-terminal thereof and a second target nucleotide sequence positioned in the vicinity of 5'-terminal thereof, under the conditions that permit an appropriate elongation reaction, with;
a primer complementary to the sequence of a region of the target nucleic acid, the region being positioned on the 5'-terminal side relative to the 5'-terminal of the first target nucleotide sequence,
a stopper nucleic acid complementary to the sequence of a region of the target nucleic acid, the region being positioned on the 3'-terminal side relative to the 3'-terminal of the second target nucleotide sequence, and the stopper nucleic acid including a modification incapable of elongation at the 3'-terminal thereof, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to a fifth aspect of the present invention, there is provided a method of preparing a detection target structure useful for detecting the target nucleotide sequence, comprising:
reacting a target nucleic acid which contains a first target nucleotide sequence positioned in the vicinity of 3'-terminal thereof, a second target nucleotide sequence positioned on the 5'-terminal side relative to the first target nucleotide sequence, a third target nucleotide sequence positioned on the 5'-terminal side relative to the second target nucleotide sequence, and a fourth target nucleotide sequence positioned in the vicinity of 5'-terminal thereof, under the conditions that permit an appropriate elongation reaction, with;
first, second and third primers complementary to the sequences of regions of the target nucleic acid, each of the regions being positioned on the 5'-terminal side relative to the 5'-terminal of each of first, second and third target nucleotide sequences
first, second and third stopper nucleic acids complementary to the sequences of regions of the target nucleic acid, each of the regions being positioned on the 3'-terminal side relative to the 3'-terminal of each of the second, third and fourth target nucleotide sequences, and the stopper nucleic acids including a modification incapable of elongation on the 3'-terminal thereof, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to a sixth aspect of the present invention, there is provided a method of preparing a detection target structure useful for detecting the target nucleotide sequence, comprising:
(1) reacting a double stranded nucleic acid which comprises a target nucleic acid having a target nucleotide sequence in the vicinity of 3'-terminal thereof and a complementary strand of the target nucleic acid, under the conditions that permit an appropriate amplification reaction, with;
   a first primer including at least a sequence which is formed of a nucleic acid not resistant to a nuclease enzyme and complementary to a target nucleotide sequence and another sequence which is formed of a nucleic acid resistant to a nuclease enzyme and complementary to a region of the target nucleic acid, the region being positioned on the 5'-terminal side relative to the 5'-terminal of the target nucleotide sequence,
   a second primer which is complementary to the sequence in the vicinity of the 5'-terminal of the target nucleic acid and contains a nucleic acid resistant to a nuclease enzyme at the 5'-terminal, and
   an enzyme having a nucleotide elongation activity; and
(2) digesting the amplified product obtained by the reaction in step (1) with a nuclease enzyme capable of decomposing the nucleic acid not resistant to the nuclease enzyme;
   thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to a seventh aspect of the present invention, there is provided a method of preparing a detection target structure useful for detecting the target nucleotide sequence, comprising:
(1) reacting a double stranded nucleic acid comprising a first target nucleic acid which has a first target nucleotide sequence in the vicinity of 3'-terminal thereof and a second target nucleic acid which is complementary to the first target nucleic acid and has a second target nucleotide sequence in the vicinity of 5'-terminal thereof, under the conditions that permit an appropriate amplification reaction, with;
   a first primer including at least a sequence which is formed of a nucleic acid not resistant to a nuclease enzyme and complementary to the first target nucleotide sequence and another sequence which is formed of a nucleic acid resistant to a nuclease enzyme and complementary to a region of the first target nucleic acid, the region being positioned on the 5'-terminal side relative to the 5'-terminal of the first target nucleotide sequence,
   a second primer including at least a sequence which is formed of a nucleic acid not resistant to a nuclease enzyme and complementary to the second target nucleotide sequence and another sequence which is formed of a nucleic acid resistant to a nuclease enzyme and complementary to a region of the second target nucleic acid, the region being positioned on the 5'-terminal side relative to the 5'-terminal of the second target nucleotide sequence, and
   an enzyme having a nucleotide elongation activity; and
(2) digesting the amplified product obtained by the reaction in step (1) with a nuclease enzyme;
   thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

According to an eighth aspect of the present invention, there is provided a method of detecting a target nucleotide sequence, comprising:
(1) preparing a detection target structure on the basis of the target nucleic acid contained in a sample by employing the method defined in any one of claims 1 to 7;
(2) allowing a nucleic acid probe complementary to the target nucleotide sequence to react with the detection target structure prepared in step (1) under the conditions that permit an appropriate hybridization; and
(3) detecting the hybridization occurred by the reaction in step (2) so as to determine the presence of the target nucleotide sequence.

According to a ninth aspect of the present invention, there is provided a detection target structure prepared by the method defined in any one of the first to seventh aspects given above.

Further, according to a tenth aspect of the present invention, there is provided an assay kit for detecting a target nucleotide sequence by performing the detection method defined in the eighth aspect given above, comprising at least one constituting element selected from the group consisting of a primer, an enzyme having a nucleotide elongation activity, a stopper nucleic acid, an enzyme for amplifying a nucleic acid, a nuclease enzyme, and the detection target structure defined in the ninth aspect given above.

### Brief Description of Drawings

FIG. 1 schematically shows the outline of the present invention;
FIG. 2 shows a first example according to a first embodiment of the present invention;
FIG. 3 shows a second example according to the first embodiment of the present invention;
FIG. 4 shows a third example according to the first embodiment of the present invention;
FIG. 5 shows a fourth example according to the first embodiment of the present invention;
FIG. 6 shows a fifth example according to the first embodiment of the present invention;
FIG. 7 shows a sixth example according to the first embodiment of the present invention;
FIG. 8 shows a seventh example according to the first embodiment of the present invention;
FIG. 9 shows an eighth example according to the first embodiment of the present invention;
FIG. 10 shows a ninth example according to the first embodiment of the present invention;
FIG. 11 shows a tenth example according to the first embodiment of the present invention;
FIG. 12 shows an eleventh example according to the first embodiment of the present invention;
FIG. 13 is a graph showing the result of the detection of the target nucleotide sequence performed in Example 1 by using a detection target structure prepared by the method of the present invention;
FIG. 14 is a graph showing the result of the detection of the target nucleotide sequence performed in Example 2 by using a detection target structure prepared by the method of the present invention;
FIG. 15 shows an example of the present invention used in Example 5;
FIG. 16 shows an example of the present invention used in Example 6; and
FIG. 17 is a graph showing the result of the detection of the target nucleotide sequence performed in Example 7 by using a detection target structure prepared by the method of the present invention.

### Best Mode for Carrying Out the Invention

### 1. Overview of the invention:

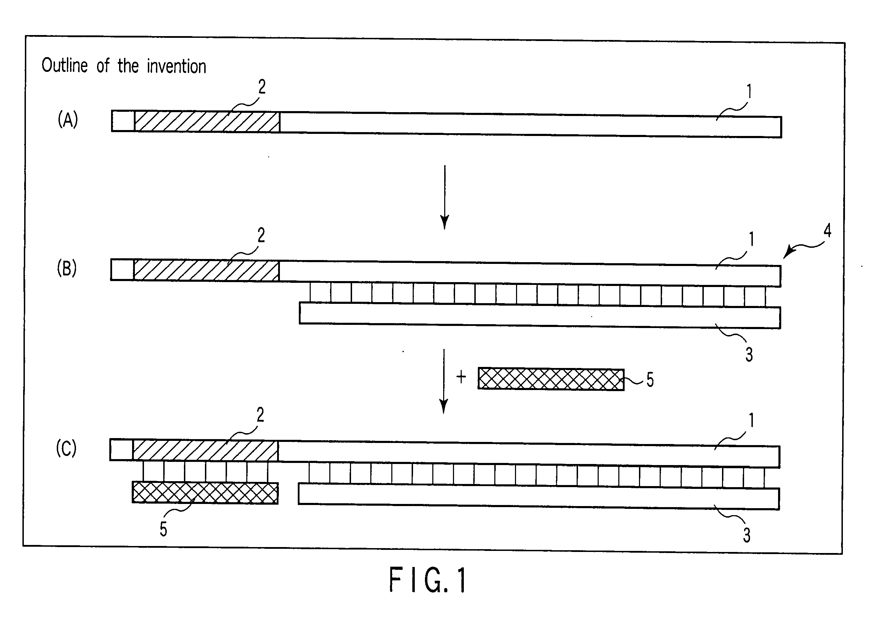

According to an embodiment of the present invention, there is provided a method of preparing a detection target structure from a target nucleic acid 1 shown in FIG. 1. The target nucleic acid 1 includes in a part a target nucleotide sequence 2 as shown in FIG. 1(A). To be more specific, according to the method of the present invention, the nucleic acid is elongated by using a primer so as to permit the target nucleic acid 1 except the portion of the target nucleotide sequence 2 to be of a double stranded structure so as to prepare a detection target structure 4, as shown in FIG. 1(B). In other words, the present invention basically provides a method of preparing a detection target structure in which an elongation reaction or an amplification reaction is carried out by using as a template the target nucleic acid containing the target nucleotide sequence 2. The present invention also provides the detection target structure 4 prepared by the preparing method of the detection target structure according to an embodiment of the present invention. Also, it is possible to detect the target nucleotide sequence by utilizing the detection target structure according to an embodiment of the present invention. In the particular detection method, a nucleic acid probe 5 complementary to the target nucleotide sequence 2 of the detection target structure 4 is allowed to react with the target nucleotide sequence 2. In this case, it is possible to determine whether the target nucleotide sequence per se or the target nucleic acid containing the target nucleotide sequence is present in the sample. The presence of the target nucleotide sequence or the target nucleic acid in the sample is detected by detecting the hybridization resulting from the reaction between the nucleic acid probe 5 and the target nucleotide sequence 2. The particular detection method of the target nucleotide sequence also falls within the scope of the present invention.

### 2. Explanation of the terms:

The term "sample" used herein is not particularly limited as far as the sample contains a nucleic acid. It suffices for the sample to be prepared by any of the known means such as the extraction, the amplification and the refining, as required, from the specimen including, for example, a tissue or cells collected from the eukaryote, including the human being, the procaryote, virus and viroid. It also suffices for the sample to be an artificially synthesized nucleic acid molecule.

The term "target nucleotide sequence" used herein denotes a single consecutive sequence that is to be detected. The term "target nucleic acid" denotes a nucleic acid containing the target nucleotide sequence. It is possible for the target nucleic acid to contain a plurality of target nucleotide sequences. The term "nucleic acid" used herein collectively denotes the substance having a structure a part of which can be generally represented by the base sequence, the particular substance including, for example, nucleic acids such as DNA, RNA, S-oligo, methyl phosphate oligo, PNA (peptide nucleic acid) and LNA (locked nucleic acid), nucleic acid analogues, nucleic acids containing some of these nucleic acids and nucleic acid analogues, and a mixture thereof. It is possible for the nucleic acid used in the present invention to be a nucleic acid present in nature, to be an artificially synthesized nucleic acid, or to be a mixture thereof. The term "nucleotide" used herein denotes a unit corresponding to a single base, which constitutes the nucleic acid referred to above. Also, the term "oligonucleotide" used herein denotes a fraction of an artificially synthesized nucleic acid referred to above, and the term "oligo DNA" denotes the DNA fraction of the artificially synthesized nucleic acid.

The term "detection target structure" used herein refers to the product particularly prepared for the purpose of detecting the target nucleotide sequence, the detection being carried out by utilizing the hybridization between the target nucleotide sequence and the nucleic acid probe corresponding to the target nucleotide sequence, and the product being prepared on the basis of the original target nucleic acid contained in the sample for the actual reaction with the nucleic acid probe. More specifically, the detection target structure according to an embodiment of the present invention includes a single stranded nucleic acid portion and a double stranded nucleic acid portion. The target nucleotide sequence is contained in the single stranded nucleic acid portion, while the double stranded nucleic acid portion comprises the target nucleic acid excluding at least the target nucleotide sequence, and a protective strand complementary thereto. In other words, the detection target structure provided in accordance with the present invention represents a nucleic acid having a specified structure including a single stranded nucleic acid portion corresponding to the target nucleotide sequence and a double stranded nucleic acid portion excluding the target nucleotide sequence. Also, the particular detection target structure can also be called a structured target nucleic acid for detection or a structured target nucleic acid.

The expressions "to elongate" and "to carry out an elongation reaction" used herein are synonymous to each other in meaning and imply that an nucleic acid is elongated by using a primer and a nucleic acid synthase enzyme under the conditions appropriate for the nucleic acid acting as a template so as to form a complementary strand of the template nucleic acid. Also, the expressions "to amplify" and "to carry out an amplifying reaction" are synonymous to each other in meaning, and imply the reactions in which a single stranded nucleic acid is elongated by using a pair of primer and nucleic acid synthase enzyme under the conditions appropriate for the nucleic acid acting as a template so as to form a complementary strand of the template nucleic acid, thereby imparting a double strand, then, the resultant double strand is denatured or subjected to strand substitution reaction so as to afford two single strands, and then, the nucleic acid elongation is conducted again by using a pair of primer and nucleic acid synthase enzyme under the appropriate conditions with the resultant two single strands used as a template so as to form a complementary strand of the template nucleic acid, thereby imparting two double strands. In other words, the term "amplification" denotes that the elongation reaction is repeatedly carried out at least twice while carrying out the denaturation between the adjacent elongation reactions.

The term "conditions under which the appropriate elongation can be attained" as used herein, denote the conditions wherein a nucleic acid to be elongated, a primer, an enzyme having a nucleotide elongation activity, substrate for nucleic acid synthesis to be incorporated into an elongated product during the elongation stage, and any coenzyme required for the elongation reaction are present together, and various conditions such as the temperature and the pH value can be controlled appropriately so as to permit a desired nucleic acid elongation. Similarly, the term "conditions under which the appropriate amplification can be attained" as used herein, denote the conditions wherein a nucleic acid to be amplified and a pair of primer, an enzyme having a nucleotide elongation activity, substrate for nucleic acid synthesis to be incorporated into an elongated product during the elongation stage, and a coenzyme required for the elongation reaction are present together, and various conditions such as the temperature and the pH value can be controlled appropriately so as to permit a desired nucleic acid elongation be repeatedly carried out at least twice, thereby enabling amplification of a desired nucleic acid.

The term "protective strand" used herein denotes a nucleic acid that is formed with use of the target nucleic acid as a template in preparing the detection target structure according to an embodiment of the present invention, the protective strand having a sequence complementary to the sequence of the target nucleic acid in a portion other than the target nucleotide sequence. The presence of the protective strand makes it possible to prevent a nucleic acid probe, another nucleic acid or the target nucleic acid itself from being hybridized with the portion other than the target nucleotide sequence, thereby preventing an unexpected formation of a double strand structure or a higher order structure.

The expressions "to detect hybrid" and "to detect association" are synonymous to each other in meaning, and denotes that, where reactions are carried out in respect of a plurality of nucleic acids under the conditions to permit an appropriate hybridization, the obtained hybrid or associated nucleic acid is detected if at least a set of complementary strands are present in the plural nucleic acids. For example, it is possible for such a hybrid or associated nucleic acid to be detected by the detection of a marker substance imparted in advance to at least one of the complementary strands or to be detected by utilizing an intercalating agent that is selectively coupled with the double stranded hybrid. On the other hand, even where the reaction is carried out in respect of a plurality of nucleic acids under the conditions that permit an appropriate hybridization, the hybrid cannot be obtained unless a complementary strand is present in the plural nucleic acids. The conditions that permit an appropriate hybridization mentioned above imply the conditions under which hybridization can be achieved by carrying out the reaction if nucleic acid strands complementary to each other are present in a certain reaction system. One example of the condition is a stringent condition.

### 3. Preparing method of detection target structure:

### (I) Method of preparing a desired detection target structure by controlling the annealing position of primer:

First to third embodiments given below are directed to a method of preparing a detection target structure for a target nucleic acid containing a target nucleotide sequence in the vicinity of the 3'-terminal. These embodiments are directed to a method of controlling the annealing position of the primer with the target nucleic acid.

### (1) First Embodiment:

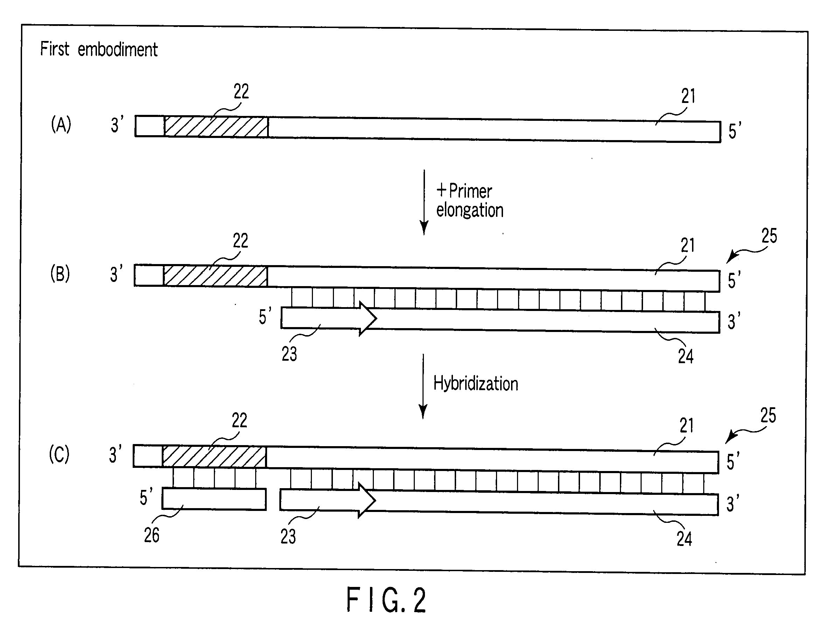

A first embodiment of a method for preparing a detection target structure according to the present invention will now be described with reference to FIG. 2. As shown in FIG. 2(A), a target nucleic acid 21 contains a target nucleotide sequence 22 in the vicinity of the 3'-terminal. A primer 23 is hybridized with the sequence on the 5'-terminal side relative to the position of the target nucleotide sequence 22 in the target nucleic acid 21 (i.e., on the 5'-terminal side relative to the 5'-terminal of the target nucleotide sequence), and the nucleic acid is elongated under the conditions that permit an appropriate elongation reaction, as shown in FIG. 2(B). As a result, formed is a protective strand 24 complementary to the target nucleic acid 21 so as to obtain a detection target structure 25 including a double strand portion and a single strand portion which contains the target nucleotide sequence, as shown in FIG. 2(B).

The detection target structure 25 obtained by the reaction according to an embodiment of the present invention can be utilized for detecting the target nucleotide sequence by using a nucleic acid probe 26, as shown in FIG. 2(C). It is possible to detect the target nucleotide sequence 22 or the target nucleic acid 21 by detecting the coupling of the nucleic acid probe 26 with the target nucleotide sequence 22.

Any kind of nucleic acid can be used as the primer. It suffices for the primer nucleic acid to be long enough to obtain hybridization specific to the sequence of the primer selected by the researcher. For example, the length of the primer nucleic acid that permits the specific hybridization should be not shorter than the length of 5 bases, preferably not shorter than the length of 9 bases.

It suffices for the elongation reaction to be carried out under the conditions that permit elongation of the primer 23 to synthesize a nucleic acid complementary to the target nucleic acid acting as a template so as to form a double stranded structure including the protective strand 24. To be more specific, it suffices for the elongation reaction to be carried out under the conditions in which an enzyme having a nucleotide synthetase activity, nucleotide substrates that is incorporated into an elongating nucleic acid during the elongation step, and a coenzyme required for the elongation reaction are present together, and in which the various conditions such as the temperature and the pH value can be controlled appropriately. It suffices for the enzyme used in the present invention to be, for example, an enzyme having an activity to elongate the nucleic acid strand from the 5'-terminal toward the 3'-terminal. The activity to elongate the nucleic acid strand is abbreviated in some cases to "nucleotide elongation activity" in the following description. It is possible to use, for example, any kind of the nucleic acid synthetase enzyme including polymerase such as DNA polymerase or RNA polymerase and a reverse transcriptase. Also, it is possible to control the temperature appropriately by using a known means such as a heater to the level selected by the researcher on the basis of, for example, the sequences of the target nucleic acid 21 and the primer 23 and the kind of the enzyme used. It suffices to control the pH value by a known means on the basis of the kinds, combinations and concentrations of the salts such as NaCl, KCl, MgCl₂ and so on. The nucleotide substrates that are incorporated into the nucleic acid during the elongation reaction stage may be the known mono-nucleotides that are generally used. It is possible for the detailed conditions to be selected by the operator in accordance with, for example, the enzyme used, the sequence and the kind of the nucleic acid.

The target nucleotide sequence 22 that can be used in the present invention may be a sequence that is to be detected by the researcher and to have a length equal to the length of 5 to 300 bases, preferably equal to the length of 9 to 50 bases.

The expression "in the vicinity of the 3'-terminal" used herein implies the bases ranging between the base positioned closest to the 3'-terminal of the target nucleic acid and the A-th base therefrom. Also, the expression "in the vicinity of the 5'-terminal" used herein later implies the bases ranging between the base positioned at the 5'-terminal and about the A-th base. For example, where the target nucleotide sequence is present in the vicinity of the 3'-terminal, it is possible for the target nucleotide sequence to include or not to include the base positioned closest to the 3'-terminal. The expression "A-th" referred to above implies the length that is half the number of bases included in the target nucleotide sequence in question, as given below:
A = (the number of bases included in the target nucleotide sequence)/2

Preferably, the method according to an embodiment of the present invention described above can be performed as follows. To be more specific, a primer and a nucleic acid synthetase enzyme are added to a sample containing the target nucleic acid so as to carry out an elongation reaction with the target nucleic acid used as a template. The primer noted above is formed of a nucleic acid having a sequence complementary to the sequence of at least 9 bases positioned adjacent to the 5'-terminal of the target nucleotide sequence that is present in the vicinity of the target nucleic acid or positioned on the 5'-terminal side relative to the target nucleotide sequence. With this particular elongation reaction, it is possible to manufacture the detection target structure including a single strand nucleic acid portion and a double strand nucleic acid portion.

In the case of preparing the detection target structure, it is necessary for the sample to contain a target nucleic acid. However, it is not absolutely necessary for the sample to contain the target nucleic acid alone. It is possible for the target nucleic acid to be contained in the sample together with another nucleic acid. In order to carry out the reaction according to an embodiment of the present invention, it is desirable for the target nucleic acid to be contained in the sample in an amount not smaller than about 3%, preferably not smaller than 10%, and more preferably not smaller than 20%, of all the nucleic acids contained in the sample. Also, it is possible to amplify the target nucleic acid by any of known amplifying means before the target nucleic acid is subjected to the method according to the embodiment of the present invention. The amplifying means that can be employed in the present invention include, for example, PCR amplification, nucleic acid strand amplification (NASBA), transcription mediated amplification (TMA), ligase strand reaction (LCR), strand displacement amplification (SDA), isothermal and chimeric primer-initiated amplification of nucleic acids (ICAN), a rolling circle amplification (RCA) method, and a loop mediated amplification (LAMP) method.

In the embodiment of the present invention described above, the boundary between the single strand nucleic acid portion and the double strand nucleic acid portion of the detection target structure that is obtained as a result of the elongation reaction is determined by the annealing position of the primer with the target nucleic acid. In the obtained detection target structure, it is possible for a single stranded nucleotide to be present on both sides of the target nucleotide sequence in a manner to cover up to about 100 bases. However, it is desirable for the target nucleotide sequence alone to form a single stranded structure.

By the method according to an embodiment of the present invention described above, it is possible to design the target nucleotide sequence freely in a smaller number of process steps. In addition, the detection target structure can be prepared easily in a short time. It follows that the economic advantage and the operability can be improved.

### (2) Second Embodiment

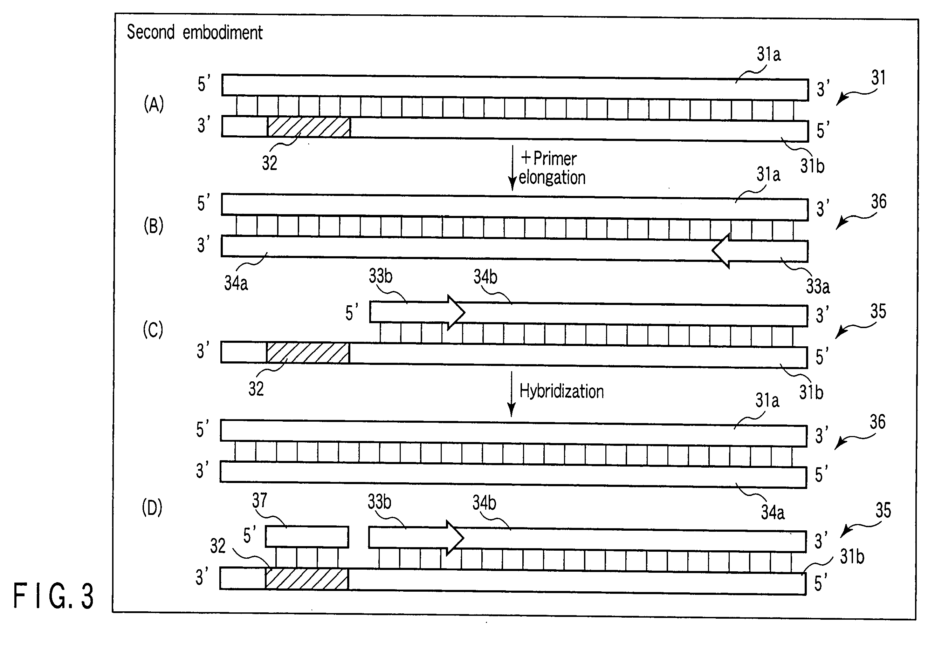

A second embodiment of the preparing method of the detection target structure according to the present invention will now be described with reference to FIG. 3. In the first embodiment described above, the target nucleic acid is of a single stranded structure. However, the second embodiment is directed to a case where the target nucleic acid is of a double stranded structure.

Attention should be paid to FIG. 3. In the first step, prepared is a target nucleic acid 31 of a double stranded structure. The target nucleic acid 31 includes a first target nucleic acid 31a and a second target nucleic acid 31b having a target nucleotide sequence 32 positioned in the vicinity of the 3'-terminal as shown in FIG. 3(A).

A first primer 33a and a second primer 33b are added to the target nucleic acid 31 so as to carry out an elongation reaction under the conditions that permit obtaining an appropriate elongation, as shown in FIGS. 3(B) and 3(C). As a result, it is possible to obtain a double stranded structure 36 (FIG. 3(B)) resulting from the first elongation, and a detection target structure 35 (FIG. 3(C)) resulting from the second elongation. The detection target structure 35 thus obtained includes a single stranded nucleic acid portion containing a target nucleotide sequence 32 and a double stranded nucleic acid portion containing a second target nucleic acid 31b that does not contain the target nucleotide sequence and a protective strand 34b.

In order to obtain an appropriate elongation in the second embodiment, it is necessary for the primers 33a and 33b to be annealed appropriately with the target nucleic acid 31. For example, it is possible to dissociate the target nucleic acid 31 of the double strand structure into a single strand structure by a known denaturing means such as heating and an addition of an alkali or a salt prior to the elongation.

The second embodiment according to the present invention described above can be worked as in the first embodiment, except that the target nucleic acid used in the second embodiment is of a double stranded structure. Also, the reacting conditions and the constructions of the target nucleic acid, the primer and the detection target structure, which are employed in the second embodiment, can be changed as in the first embodiment.

It is possible to utilize the detection target structure 35 obtained by the elongation reaction for the detection of the target nucleotide sequence using a nucleic acid probe 37, as shown in FIG. 3(D). For example, it is possible to detect the target nucleotide sequence by detecting the hybridization of the nucleic acid probe 36 with the target nucleotide sequence 32 as in the method of the first embodiment.

By the method according to the embodiment of the present invention described above, it is possible to design the target nucleotide sequence freely in a smaller number of process steps. In addition, the detection target structure can be prepared easily in a short time. It follows that the economic advantage and the operability can be improved.

### (3) Third Embodiment

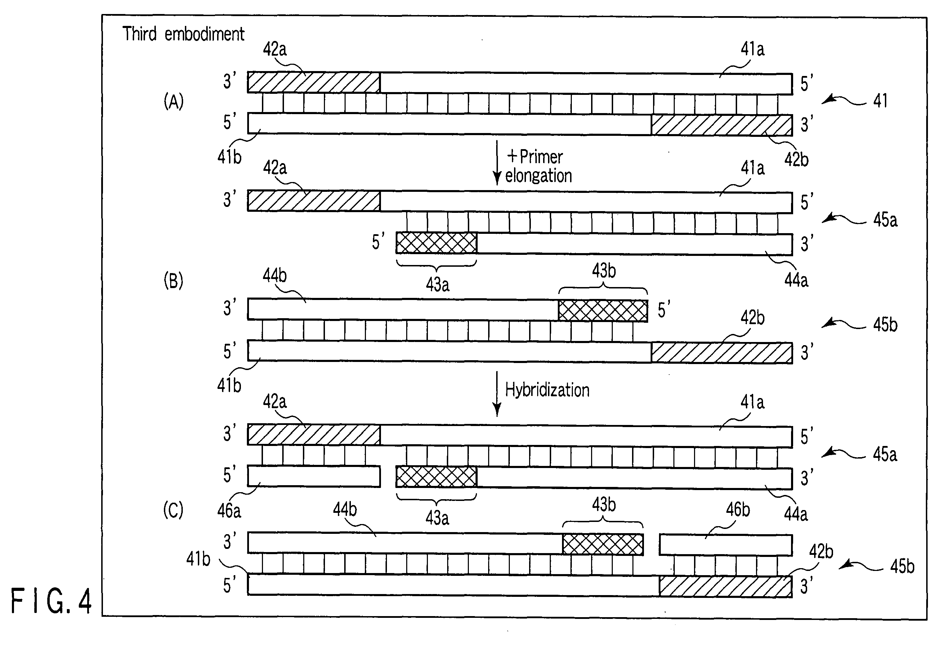

A third embodiment of the preparing method of a detection target structure according to the present invention will now be described with reference to FIG. 4. The third embodiment can be worked by the procedure equal to that in the second embodiment, except that, in the second embodiment described above, a single detection target structure 35 is obtained from the target nucleic acid 31 of a double stranded structure, whereas, in the third embodiment, two detection target structures 45a and 45b are obtained from a nucleic acid 41 of a double stranded structure.

Attention should be paid to FIG. 4. The target nucleic acid 41 of a double stranded structure includes a first target nucleic acid 41a containing a first target nucleotide sequence 42a positioned in the vicinity of the 3'-terminal, and a second target nucleic acid 41b containing a second target nucleotide sequence 42b positioned in the vicinity of the 3'-terminal, as shown in FIG. 4(A).

A first primer 43a and a second primer 43b are added to the target nucleic acid 41 so as to carry out an elongation reaction of the nucleic acid under the conditions that permit obtaining an appropriate elongation, as shown in FIG. 4(B). As a result, it is possible to obtain a first target structure 45a resulting from the first elongation and a second detection target 45b resulting from the second elongation, as shown in FIG. 4(B). Each of the detection target structures 45a and 45b, each of which includes a single stranded nucleic acid portion containing a target nucleotide sequence 42a or 42b and a double stranded nucleic acid portion containing a portion of the target nucleotide acid 41a or 41b and a protective strand 44a or 44b complementary thereto.

The third embodiment according to the present invention described above can be worked as in the method of the second embodiment; except that two detection target structures can be obtained in the third embodiment from the double stranded target nucleic acid. It follows that it suffices for the conditions under which an appropriate elongation can be obtained in the third embodiment, the reacting conditions employed in the third embodiment, and the condition for the target nucleic acid, the primer and the detection target structure to be substantially equal to those described previously in conjunction with the second embodiment. It is also possible to modify the third embodiment in various fashions as in the second embodiment.

It is possible to utilize the detection target structure 45a and/or the detection target structure 45b for the detection of the target nucleotide sequence using the nucleic acid probe 46a and/or the nucleic acid probe 46b, as shown in FIG. 4(C). For example, it is possible to detect the target nucleotide sequence by detecting the hybridization of the nucleic acid probe 46a and/or 46b with the target nucleotide sequence 42a and/or 42b.

By the method according to the embodiment of the present invention described above, it is possible to design the target nucleotide sequence freely in a smaller number of process steps. In addition, the detection target structure can be prepared easily in a short time. It follows that the economic advantage and the operability can be improved. Further, it is possible to detect simultaneously the target nucleotide sequence at two points in accordance with the third embodiment.

### (II) Method using stopper nucleic acid:

Forth to sixth embodiments described in the following are directed to the method of preparing a detection target structure on the basis of a target nucleic acid containing a target nucleotide sequence in the vicinity of the 5'-terminal.

### (4) Fourth Embodiment:

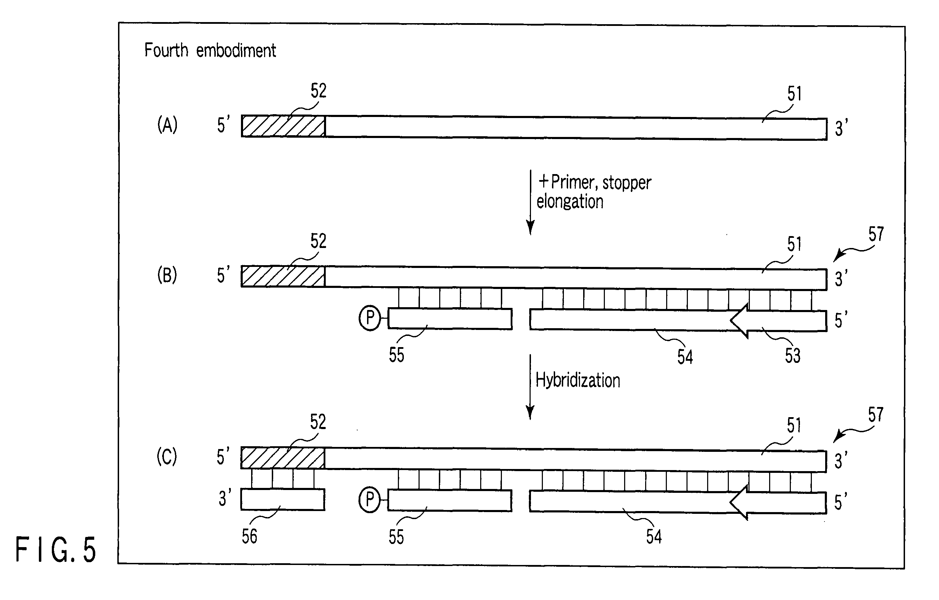

A forth embodiment of the method of preparing a detection target structure according to an embodiment of the present invention will now be described with reference to FIG. 5. As shown in FIG. 5(A), a target nucleic acid 51 contains a target nucleotide sequence 52 in the vicinity of the 5'-terminal. In the first step, selected is a sequence for allowing a primer 53 to be annealed in the vicinity of the 3'-terminal of the target nucleic acid 51. Also selected is a sequence for allowing a stopper nucleic acid 55 to be annealed on the 3'-terminal side relative to the position of the target nucleotide sequence 52 in the target nucleic acid 51. In order to prevent the elongation reaction from further proceeding toward the 3'-terminal of the stopper nucleic acid 55, a modification that inhibits the elongation is applied to the 3'-terminal of the stopper nucleic acid 55.

The primer 53 annealed to the sequence in the vicinity of the 3'-terminal of the target nucleic acid 51 is elongated from the 5'-terminal thereof toward the 3'-terminal thereof under the conditions that permit appropriate elongation reaction, under the state that the stopper nucleic acid 55 is annealed in the position on the 3'-terminal side relative to the target nucleotide sequence 52 in the target nucleic acid 51. As a result, a protective strand 54 is formed as shown in FIG. 5(B). As described above, the 3'-terminal of the stopper nucleic acid 55 is modified so as not to be further subjected to the elongation reaction. As a result, the elongation reaction does not further proceed beyond the stopper nucleic acid 55. It follows that the target nucleotide sequence 52 included in the target nucleic acid 51 is retained in the form of a single stranded structure.

The primer 53 used in the fourth embodiment may be any kind of nucleic acid. Also, it suffices for the primer nucleic acid to have sufficient length for obtaining the specific hybridization to the sequence selected by the researcher. For example, the length of the primer nucleic acid, which permits obtaining the specific hybridization, is as described previously in conjunction with the first embodiment.

It is possible to use any kind of nucleic acid as the stopper nucleic acid in this embodiment, and it suffices for the stopper nucleic acid to have a sufficient length for annealing on the sequence at a desired position of the target nucleic acid. For example, the length of the stopper nucleic acid that permits achieving the specific annealing should be equal to the length of at least 5 bases, preferably equal to the length of at least 9 bases. The modification at the 3'-terminal of the stopper nucleic acid is not particularly limited as long as the elongation reaction does not proceed further toward the 3'-terminal. The modification at the 3'-terminal can be achieved by, for example, the phosphorylation and the use of dideoxy nucleotide, but not limited thereto. Also, it is possible to select the kind of the modification in accordance with the kind of enzyme used that has the nucleotide elongation activity. FIG. 5 exemplifies the modification of the 3'-terminal of the stopper nucleic acid by means of phosphorylation. The particular modification is denoted by the mark P in the drawing.

It suffices for the elongation reaction that can be employed in this embodiment to be carried out under the conditions that permit the primer 53 to start the elongation of the nucleic acid with the target nucleic acid acting as a template so as to form a double stranded structure including the protective strand 52. In other words, it suffices for the elongation reaction to be carried out under the conditions in which, for example, an enzyme having a nucleotide elongation activity, a mono-nucleotide units for nucleic acid synthesis that is incorporated in the nucleic acid during the elongation, and a coenzyme required for the elongation reaction are present together, and in which the various conditions such as the temperature and the pH value can be controlled appropriately. The enzyme that can be used in the present invention is as already described in conjunction with the first embodiment. Also, the appropriate temperature control and the pH value control are also as already described in conjunction with the first embodiment.

The target nucleotide sequence 52 that can be used in the present invention is not particularly limited as long as the researcher wishes to detect the particular sequence. Also, it suffices for the target nucleotide sequence 52 to have a length equal to the length of 5 bases to 300 bases, preferably equal to the length of 9 bases to 50 bases. Further, it is possible for the target nucleotide sequence 52, which is present in the vicinity of the 5'-terminal of the target nucleic acid 51, to include or not to include the base comprising the 5'-terminal.

It is desirable for the method according to the embodiment of the present invention to be worked as follows. Specifically, a stopper nucleic acid, a primer formed of a nucleic acid complementary to the sequence in the vicinity of the 3'-terminal of the target nucleic acid, and a nucleic acid synthetase enzyme are added to a sample containing the target nucleic acid so as to carry out an elongation reaction with the target nucleic acid acting as a template. The stopper nucleic acid noted above is designed to have a sequence which is complementary to the sequence of at least 9 bases adjacent to the 3'-terminal of the target nucleotide sequence 52 present in the vicinity of the 5'-terminal of the target nucleic acid, or present on the 3'-terminal side relative to the target nucleotide sequence 52. In addition, the stopper nucleic acid is designed to have the 3'-terminal modified with a functional group other than the hydroxyl group (-OH). As a result of the elongation reaction referred to above, a detection target structure including a single strand nucleic acid portion and a double strand nucleic acid portion is prepared.

Also, in the method described above, the elongation reaction from the primer 53 serves to synthesize a protective strand 54 having a sequence of same polarity as that of the nucleic acid probe sequence which is complementary to the target nucleotide sequence used in the method of detecting the target nucleotide sequence.

It is possible to utilize the detection target structure 57 obtained by the embodiment described above for detecting the target nucleotide sequence 52 by using the nucleic acid probe 56, as shown in FIG. 5(C). The nucleic acid probe 56 can be hybridize with the target nucleotide sequence 52 as described previously in conjunction with the first embodiment.

In the embodiment of the present invention described above, the target nucleic acid is used as a template, and the nucleic acid elongation is achieved by using a primer and a stopper nucleic acid. To be more specific, in this embodiment, the boundary between the single stranded nucleic acid portion and the double stranded nucleic acid portion included in the detection target structure is determined because the stopping position of the elongation reaction is controlled by selecting the position where the stopper nucleic acid 55 is annealed on the target nucleic acid 51. As a result, the detection target structure is constructed such that the region of the target nucleotide sequence 52 on the target nucleic acid 51 is of a single strand and the other region is of a double strand.

By the method according to the embodiment described above, it is possible to design the target nucleotide sequence freely in a smaller number of process steps. In addition, the detection target structure can be prepared easily in a short time. It follows that the economic advantage and the operability can be improved.

### (5) Fifth Embodiment:

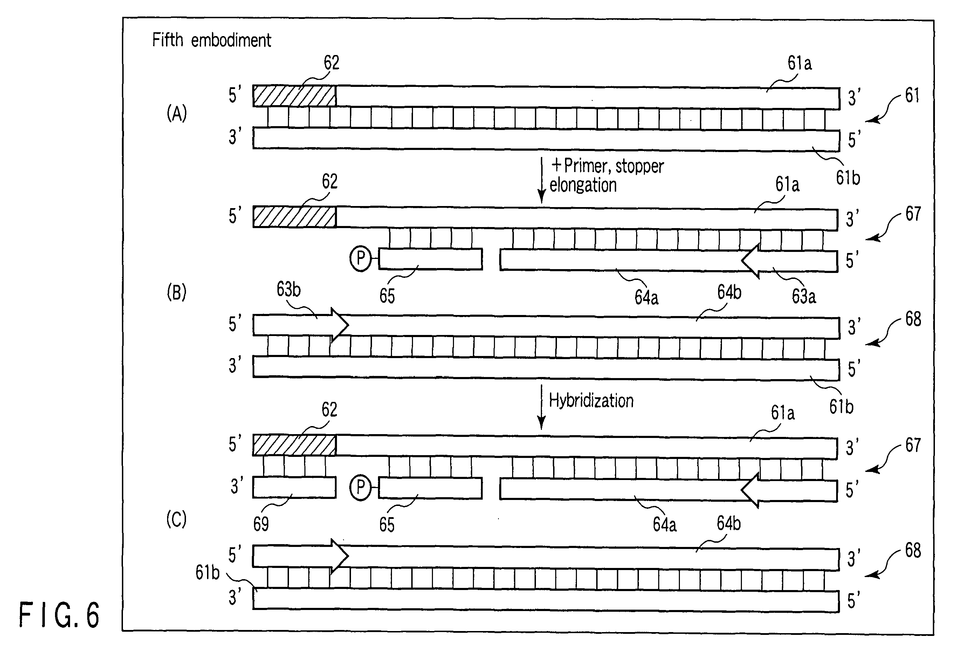

A fifth embodiment of preparing the detection target structure according to the present invention will now be described with reference to FIG. 6. In the fourth embodiment described above, the target nucleic acid 51 is of a single stranded structure. On the other hand, a double stranded nucleic acid is used in the fifth embodiment.

Attention should be paid to FIG. 6. As apparent from the drawing, prepared is a double stranded target nucleic acid 61. The double strand nucleic acid 61 includes a first target nucleic acid 61a having a target nucleotide sequence 62 in the vicinity of the 5'-terminal and a second target nucleic acid 61b, as shown in FIG. 6(A).

In the first step, a sequence for allowing a primer 63 to be annealed in the vicinity of the 3'-terminal of the target nucleic acid 61a is determined. Also determined is a sequence for allowing a stopper nucleic acid 65 to be annealed on the 3'-terminal side relative to the target nucleotide sequence 62 in the target nucleic acid 61a. The 3'-terminal of the stopper nucleic acid 65 is modified so as to prevent the elongation reaction from further proceeding toward the 3'-terminal.

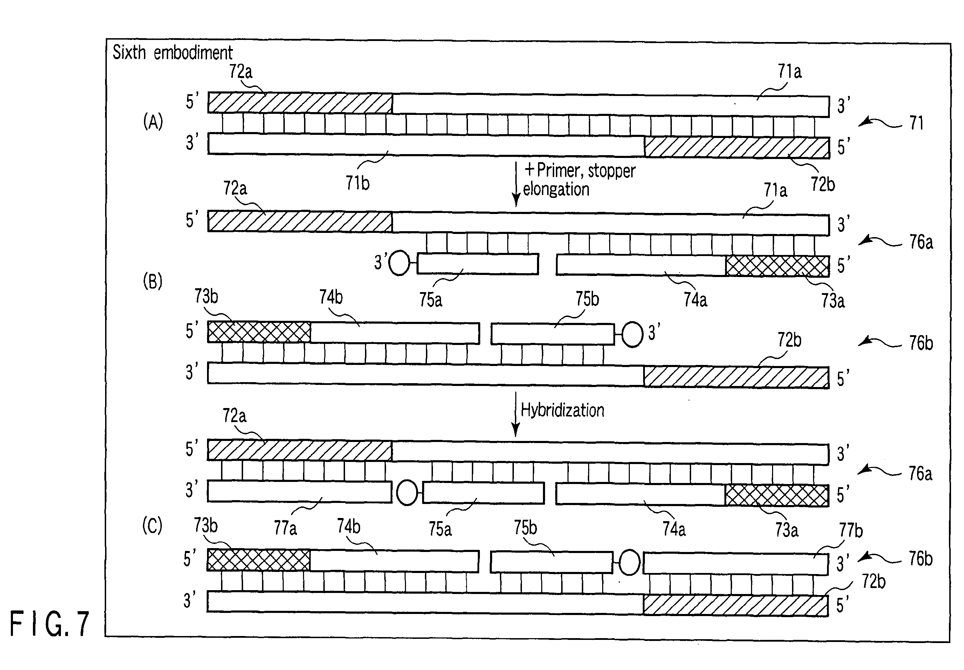

The nucleic acid is elongated from the 5'-terminal toward the 3'-terminal under the conditions that permit an appropriate elongation reaction by using a primer 63a annealed on the sequence in the vicinity of the 3'-terminal of the target nucleic acid 61a and a primer 63b annealed on the sequence in the vicinity of the 3'-terminal of the target nucleic acid 61b under the state that the stopper nucleic acid 65 is hybridized to the sequence on t 3'-terminal side relative to the target nucleotide sequence 62 in the target nucleic acid 61a, as shown in FIG. 7(B). Protective strands 64a and 64b are formed by the elongation reaction referred to above.

After the elongation reaction, obtained are a detection target structure 67 on the basis of the first target nucleic acid 61a as a first elongation product, and a double strand 68 on the basis of the second target nucleic acid 61b as a second elongation product.

In order to obtain an appropriate elongation in the fifth embodiment, it is necessary for the primers 63a and 63b to be annealed appropriately on the target nucleic acids 61a and 61b, respectively. For achieving this step, it is possible to dissociate the target nucleic acid 61 of the double strand into a single strand by a known denaturing means such as heating and an addition of an alkali or a salt prior to the elongation.

The fifth embodiment according to the present invention described above can be worked as in the fourth embodiment, except that the target nucleic acid used in the fifth embodiment is of a double strand. Also, the reacting conditions and the constructions of the target nucleic acid, the primer and the detection target structure, which are employed in the fifth embodiment, can be changed as described in the fourth embodiment.

The detection target structure 67 obtained through the elongation reaction described above can be utilized for the detection of the target nucleotide sequence, by using the nucleic acid probe 69, as shown in FIG. 6(C). It is possible to detect the target nucleotide sequence 62 by detecting the hybridization between the nucleic acid probe 69 having a sequence complementary to the target nucleotide sequence 62 and the target nucleotide sequence 62. The detection of the hybridization can be performed by the method similar to that described previously in conjunction with the first embodiment.

By the embodiment of the present invention described above, it is possible to design the target nucleotide sequence freely in a smaller number of process steps. In addition, the detection target structure can be prepared easily in a short time. It follows that the economic advantage and the operability can be improved.

### (6) Sixth Embodiment:

A sixth embodiment of preparing the detection target structure according to the present invention will now be described with reference to FIG. 7. The sixth embodiment can be worked in a similar manner as in the fifth embodiment, except that, in the fifth embodiment described above, a single detection target structure 67 is obtained from the double stranded target nucleic acid 61, whereas, in the sixth embodiment described in the following, tow detection target structures 76a and 76b are obtained from a pair of nucleic acid strand 71a and 71b, respectively, which are included in a double strand target nucleic acid 71.

Attention should be paid to FIG. 7. As apparent from the drawing, a double strand target nucleic acid 71 includes a first target nucleic acid 71a containing a first target nucleotide sequence 72a in the vicinity of the 5'-terminal and a second target nucleic acid 71b containing a second target nucleotide sequence 72b in the vicinity of the 5'-terminal.

In the first step, a sequence for allowing a primer 73a to be annealed on the vicinity of the 3'-terminal of the target nucleic acid 71a is determined. Also determined is a sequence for allowing a stopper nucleic acid 75a to be annealed on the 3'-terminal side relative to the target nucleotide sequence 72a of the target nucleic acid 71a. The 3'-terminal of the stopper nucleic acid 75a is modified so as to prevent the elongation reaction from further proceeding toward the 3'-terminal. Likewise, a sequence for allowing a primer 73b to be annealed in the vicinity of the 3'-terminal of the target nucleic acid 71b is determined. Also determined is a sequence for allowing the stopper nucleic acid 75b to be annealed on the 3'-terminal side relative to the target nucleotide sequence 72b of the target nucleic acid 71b. The 3'-terminal of the stopper nucleic acid 75b is modified so as to prevent the elongation reaction from further proceeding toward the 3'-terminal. The mark "○" in FIG. 7 indicates that each of the 3'-terminals of the stopper nucleic acids 75a and 75b are modified so as to prevent the elongation reaction from further proceeding toward the 3'-terminal.

The nucleic acid is elongated from the 5'-terminal toward the 3'-terminal by using primers 73a and 73b which are hybridized in the vicinity of the 3'-terminals of the target nucleic acids 71a and 71b, respectively, under the conditions that permit an appropriate elongation reaction under the state that the stopper nucleic acids 75a and 75b are hybridized with the sequences on the 3'-terminal side relative to the target nucleotide sequences 72a and 72b of the target nucleic acids 71a, 71b, respectively. As a result, protective strands 74a and 74b are formed so as to afford a detection target structure 76a as a first elongation product and a detection target structure 76b as a second elongation product. The portions of the target nucleotide sequence 72a and the target nucleotide sequence 72b are both retained in the form of a single stranded structure. Each of the detection target structures 76a and 76b includes a single stranded nucleic acid portion containing the target nucleotide sequences 72a and 72b and a double stranded nucleic acid portion containing portions of the target nucleic acids 71a, 71b and protective strands 74a, 74b complementary to the target nucleic acids 71a, 71b.

The sixth embodiment according to the present invention described above can be worked as described in the fifth embodiment, except that two detection target structures can be obtained in the sixth embodiment from the double strand structure constituting the target nucleic acid. It follows that it is possible for the conditions that permit an appropriate elongation in the sixth embodiment and the conditions in respect of the target nucleic acid, the stopper nucleic acid, the primer and the detection target structure to be substantially equal to those for the fifth embodiment. Also, the sixth embodiment can be modified in various fashions as described in the fifth embodiment.

Also, the detection target structure 76a and the detection target structure 76b obtained in the elongation reaction described above can be utilized for detecting the nucleic acid probe 77a and for detecting the target nucleotide sequence by using the nucleic acid probe 77b. For example, it is possible to detect the target nucleotide sequences by detecting the hybridization of the nucleic acid probes 77a and 77b with the target nucleotide sequences 72a and 72b as in any of the embodiments described above.

By the method according to the embodiment described above, it is possible to design the target nucleotide sequence freely in a smaller number of process steps. In addition, the detection target structure can be prepared easily in a short time. It follows that the economic advantage and the operability can be improved. Further, according to the sixth embodiment, it is possible detect simultaneously the target nucleotide sequences at two points.

### (IV) Method of forming a hybridization portion for a nucleic acid probe by controlling the annealing position of the primer and by using a stopper nucleic acid:

It is possible to design freely various types of target nucleotide sequences by combining the methods described under items (I) and (II) given previously. Embodiments 7 to 9 given below are directed to the particular combinations.

### (7) Seventh Embodiment:

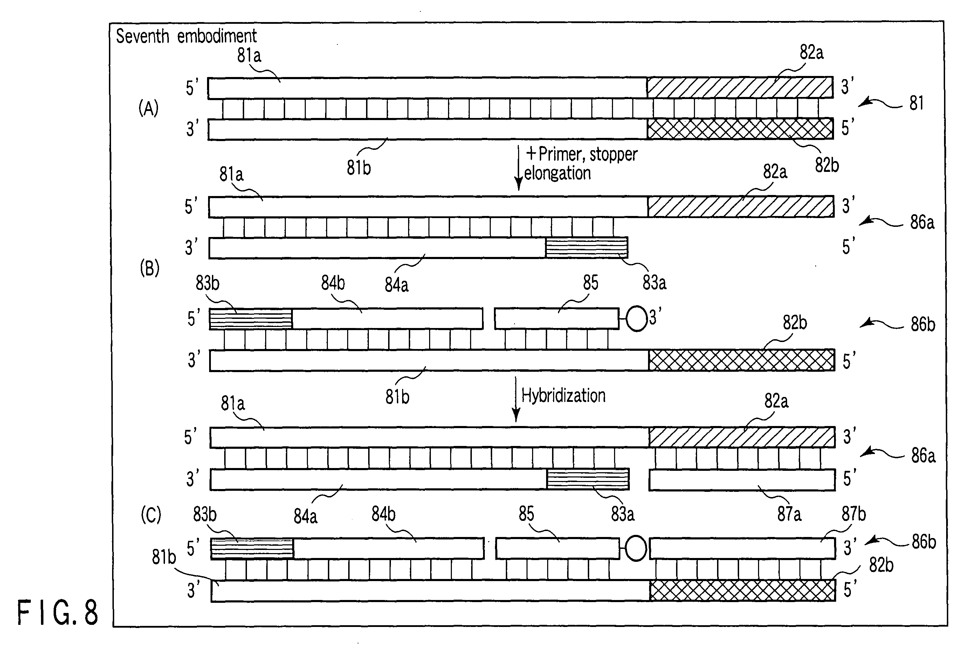

A seventh embodiment of preparing the detection target structure according the present invention will now be described with reference to FIG. 8. In the sixth embodiment described above, each of the first target nucleic acid and the second target nucleic acid includes a target nucleotide sequence in the vicinity of the 5'-terminal. In the seventh embodiment described in the following, however, the first target nucleic acid includes a target nucleotide sequence in the vicinity of the 3'-terminal, and the second target nucleic acid includes a target nucleotide sequence in the vicinity of the 5'-terminal.

Attention should be paid to FIG. 8. As shown in the drawing, a target nucleic acid 81 having a double strand structure includes a first target nucleic acid 81a and a second target nucleic acid 81b. A first target nucleotide sequence 82a is present in the vicinity of the 3'-terminal of the first target nucleic acid 81a. Likewise, a second target nucleotide sequence 82b is present in the vicinity of the 5'-terminal of the second target nucleic acid 81b. It is possible for the first target nucleotide sequence 82a and the second target nucleotide sequence 82b to be or not to be complementary to each other.

The first detection target structure 86a can be prepared from the first target nucleic acid 81a by the method similar to that described previously in conjunction with the first embodiment. To be more specific, it is possible to form a protective strand 84a by carrying out an elongation reaction by using a primer 83a having a sequence complementary to the sequence on the 5'-terminal side relative to the position of the target nucleotide sequence 82a included in the first target nucleic acid 81a. The elongation reaction referred to above is carried out under the conditions that permit obtaining an appropriate elongation so as to form the protective strand 84a and, thus, to afford a detection target structure 86a. Also, it is possible to achieve various modifications as already described in conjunction with the first embodiment.

The second target structure 86b can be prepared from the second target nucleic acid 81b by the method similar to that described previously in conjunction with the fifth embodiment. To be more specific, a protective strand 84b is formed by carrying out an elongation reaction in the presence of the stopper nucleic acid 85 by using a primer 83b complementary to a part of the sequence in the vicinity of the 3'-terminal of the second target nucleic acid, thereby preparing the detection target structure 86b. It should be noted that the stopper nucleic acid 85 has a sequence complementary to the sequence on the 3'-terminal side relative to the position of the target nucleotide sequence 82b included in the target nucleic acid 81b. The preparation of the detection target structure from the second target nucleic acid can be modified in various fashions as described previously in conjunction with the fifth embodiment. It should be noted that, in FIG. 8, the mark "○" indicates the modification at the 3'-terminal of the stopper nucleic acid 85 for inhibiting the elongation reaction proceeding toward the 3'-terminal therefrom.

The elongation reaction on the first target nucleic acid 81a and the elongation reaction on the second target nucleic acid 81b can be performed simultaneously or sequentially. For example, in the case of carrying out the elongation simultaneously, it suffices to carry out the elongation reaction under the conditions that permit an appropriate elongation reaction in respect of both the first target nucleic acid 81a and the second target nucleic acid 81b as in the other embodiments in which the target nucleic acid is of a double stranded structure.

It is possible for the first target nucleotide sequence 82a and the second target nucleotide sequence 82b to have the same length and to be 100% completely complementary to each other. It is also possible for one of the first and second target nucleotide sequences 82a, 82b to be longer than the other, or to have the same length and to have the terminals slightly misaligned from each other.

By the method according to the embodiment described above, it is possible to design the target nucleotide sequence freely in a smaller number of process steps. In addition, the detection target structure can be prepared easily in a short time. It follows that the economic advantage and the operability can be improved. Further, according to the seventh embodiment, it is possible to detect simultaneously or sequentially the target nucleotide sequences at corresponding portions on the two complementary strands. It follows that it is possible to detect without fail the desired target nucleotide sequence so as to improve the accuracy of the judgment. The particular method is advantageous in the case of detecting, for example, a single nucleotide polymorphism (SNP).

Also, the detection of the target nucleotide sequences 82a, 82b using the detection target structures 86a, 86b can be performed by detecting the hybridization between the first target nucleotide sequence 82a and the nucleic acid probe 87a complementary thereto, and the hybridization between the second target nucleotide sequence 82b and the nucleic acid probe 87b complementary to thereto, as in the other embodiments described previously.

### (8) Eighth Embodiment:

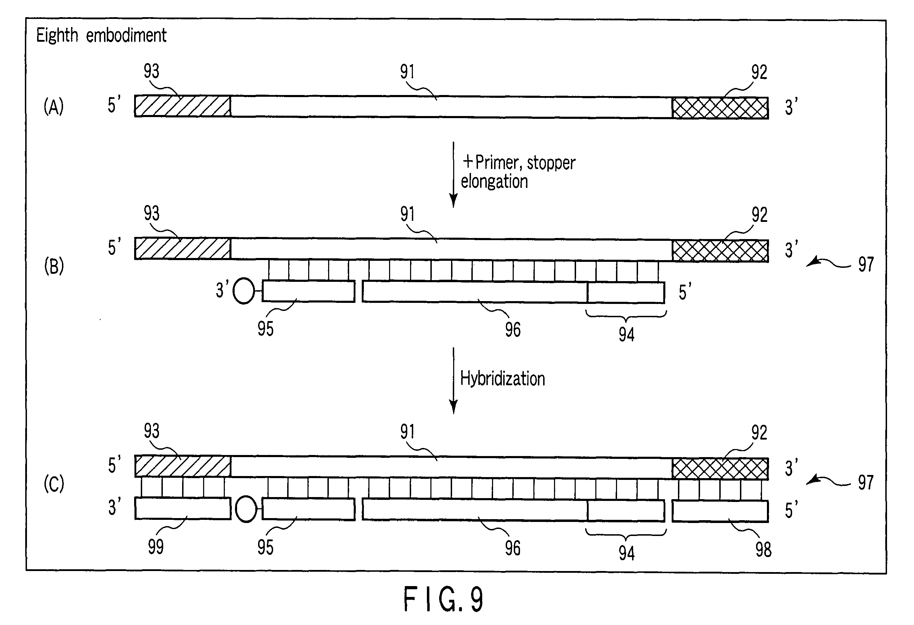

An eighth embodiment of preparing the detection target structure according to the present invention will now be described with reference to FIG. 9. Attention should be paid to FIG. 9. As shown in FIG. 9(A), a target nucleic acid 91 of a single strand structure has a first target nucleotide sequence 92 in the vicinity of the 3'-terminal and a second target nucleotide sequence 93 in the vicinity of the 5'-terminal. A detection target structure 97 is prepared from the target nucleic acid 91 of the particular construction as shown in FIG. 9(B). A primer 94 complementary to a part of the sequence is present on the 5'-terminal side relative to the position of the first target nucleotide sequence 92 included in the target nucleic acid 91. Also, a stopper nucleic acid 95 complementary to a part of the sequence is present on the 3'-terminal side relative to the position of the second target nucleotide sequence 93 included in the target nucleic acid 91. An elongation reaction is carried out under the conditions that permit an appropriate elongation reaction so as to form a protective strand 96, thereby creating a detection target structure 97. The 3'-terminal of the stopper nucleic acid 95 is modified for preventing the elongation reaction from further proceeding toward the 3'-terminal, as denoted by the mark "○".

The detection of each of the target nucleotide sequences 92, 93 using the detection target structure 97 is performed by detecting the hybridization between the first target nucleotide sequence 92 and the nucleic acid probe 98 complementary thereto, and the hybridization between the second target nucleotide sequence 93 and a second nucleic acid probe 99 complementary thereto. The hybridization noted above is detected by the method similar to that described previously in conjunction with the other embodiments.

In the eighth embodiment described above, the target nucleic acid used is of a single stranded structure. However, it is also possible to apply similarly the eighth embodiment to the target nucleic acid of a double stranded structure. Also, the eighth embodiment can be modified as desired in such a manner that described previously in conjunction with the first embodiment, the second embodiment, the fourth embodiment, and the fifth embodiment.

By the method according to the embodiment of the present invention described above, it is possible to design the target nucleotide sequence freely in a smaller number of process steps. In addition, the detection target structure can be prepared easily in a short time. It follows that the economic advantage and the operability can be improved.

### (9) Ninth Embodiment:

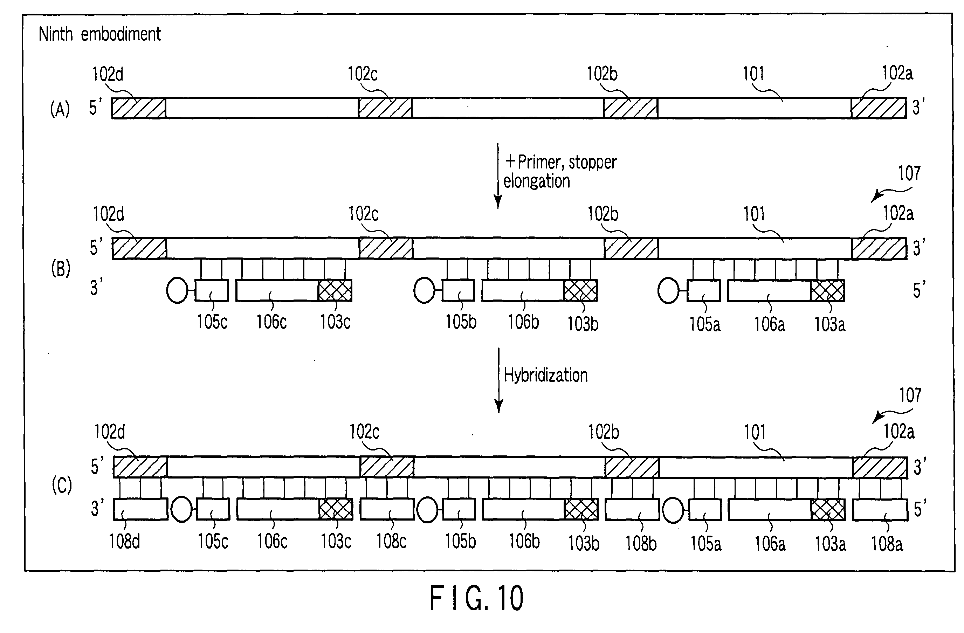

An embodiment of preparing a detection target structure for the target nucleic acid having at least three target nucleotide sequences will now be described with reference to FIG. 10. Attention should be paid to FIG. 10. As shown in FIG. 10(A), a target nucleic acid 101 of a single stranded structure comprises a first target nucleotide sequence 102a in the vicinity of the 3'-terminal, a second target nucleotide sequence 102b, a third target nucleotide sequence 102c, and a fourth target nucleotide sequence 102d which is in the vicinity of the 5'-terminal. A detection target structure is prepared from the target nucleic acid 101 of the particular construction. In the first step, prepared are a first primer 103a complementary to the sequence on the 5'-terminal side relative to the 5'-terminal of the first target nucleotide sequence 102a, a second primer 103b complementary to the sequence on the 5'-terminal side relative to the 5'-terminal of the second target nucleotide sequence 102b, and a third primer 103c complementary to the sequence on the 5'-terminal side relative to the 5'-terminal of the third target nucleotide sequence 102c. Further, also prepared are three stopper nucleic acids. A first stopper nucleic acid 105a has a sequence complementary to the sequence on the 3'-terminal side relative to the position of the second target nucleotide sequence 102b. A second stopper nucleic acid 105b has a sequence complementary to the sequence on the 3'-terminal side relative to the position of the third target nucleotide sequence c. Further, a third stopper nucleic acid 105c has a sequence complementary to the sequence on the 3'-terminal side relative to the position of the forth target nucleotide sequence 102d.

An elongation reaction is carried out under the conditions that the first primer 103a, the second primer 103b, the third primer 103c, the first stopper nucleic acid 105a, the second stopper nucleic acid 105b and the third stopper nucleic acid 105 are present together, and that an appropriate elongation reaction can be carried out. As a result of the elongation reaction, formed are a first protective strand 106a, a second protective strand 106b and a third protective strand 106c, thereby creating a detection target structure 107 as shown in FIG. 10(B).

The detection target structure 107 thus prepared can be used for detecting simultaneously the presence of the four target nucleotide sequences. The detection can be performed by detecting the hybridization of the nucleic acid probes 108a, 108b, 108c and 108d with the first target nucleotide sequence 102a, the second target nucleotide sequence 102b, the third target nucleotide sequence 102c and the fourth target nucleotide sequence 102d, respectively.

The ninth embodiment described above is directed to the case where four target nucleotide sequences are formed on a single stranded target nucleic acid. However, it is also possible to apply the method of the ninth embodiment to any case as long as at least three target nucleotide sequences are formed on the target nucleic acid. Also, it is not absolutely necessary for the target nucleotide sequences to be present in abutting against the 5'-terminal and/or the 3'-terminal of the target nucleic acid. In other words, it is possible for the target nucleotide sequence to be present at any of the terminals with some of bases interposed therebetween or to be present in only the mid portion of the target nucleic acid. Also, the 3'-terminals of the stopper nucleic acids 105a, 105b and 105c are modified for preventing the elongation reaction from further proceeding toward the 3'-terminals as denoted by the marks "○" in FIG. 10.

In the ninth embodiment described above, the target nucleic acid used is of a single stranded structure. However, it is also possible to apply similarly the method of the ninth embodiment to the target nucleic acid having a double stranded structure. In this case, it is possible to obtain a single detection target structure or two detection target structures from the target nucleic acid of a double stranded structure. Also, the ninth embodiment can be modified as desired in accordance with the methods described previously in conjunction with the first embodiment, the second embodiment, the fourth embodiment, and the fifth embodiment.

By the method according to the embodiment of the present invention described above, it is possible to design the target nucleotide sequence freely in a smaller number of process steps. In addition, the detection target structure can be prepared easily in a short time. It follows that the economic advantage and the operability can be improved.

### (V) Method of forming a nucleic acid probe coupling portion by using enzyme:

Tenth and eleventh embodiments described in the following are directed to a method that the entire length of a target nucleic acid is duplicated in the form of a double stranded structure, followed by making the desired target nucleotide sequence portion alone to be a single stranded structure. To be more specific, the sequence in at least a part of the primer used for the nucleic acid amplification is modified in advance so as to permit the sequence to be resistant to the nuclease enzyme, followed by elongating or amplifying the sample by using the primer and subsequently digesting the double strand nucleic acid resulting from the elongation or the amplification including the modified portion by using an exonuclease. As a result, it is possible to prepare a detection target structure having a single strand nucleic acid portion and a double strand nucleic acid portion.

### (10) Tenth embodiment:

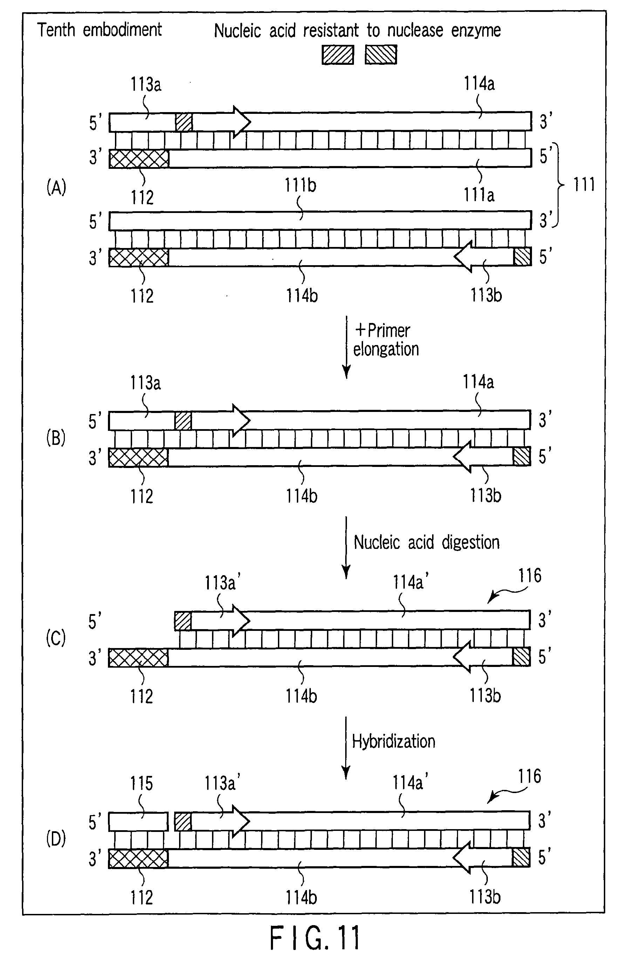

A tenth embodiment of the preparing method of the detection target structure according to an embodiment of the present invention will now be described with reference to FIG. 11. A target nucleic acid 111 providing the basis of the detection target structure is formed of a double stranded target nucleic acid consisting of a first target nucleic acid 111a and a second target nucleic acid 111b. The first target nucleic acid 111a includes a target nucleotide sequence 112 in the vicinity of the 3'-terminal. By using a first primer 113a and a second primer 113b, respectively, protective strands 114a and 114b are elongated on the target nucleic acid 111 of the particular construction.

The first primer 113a is a primer complementary to the sequence in the vicinity of the 3'-terminal of the first target nucleic acid 111a and includes a sequence complementary to the target nucleotide sequence 112 and another sequence complementary to the sequence of the target nucleic acid on the 5'-terminal side relative to the target nucleotide sequence 112. Also, the sequence portion complementary to the target nucleotide sequence 112 included in the first primer 113a is formed of nucleotides that are not resistant to the nuclease enzyme. In contrast, a part of the sequence complementary to the sequence on the 5'-terminal side relative to at least the target nucleotide sequence 112, preferably a part of the sequence complementary to the sequence adjacent to the target nucleotide sequence, is provided by a nucleic acid resistant to the nuclease enzyme. On the other hand, the second primer 113b is a primer complementary to the sequence in the vicinity of the 3'-terminal of the second target nucleic acid 111b. Since at least the 5'-terminal of the second primer 113b is resistant to the nuclease enzyme, the second primer 113b is not digested by the nuclease enzyme.

The nucleic acid elongation reaction is performed in the presence of the target nucleic acid 111, the first primer 113a and the second primer 113b under the conditions that permit carrying out an appropriate elongation so as to afford a double stranded structure, as shown in FIG. 11(A). Then, the double stranded structure thus obtained is denatured to give two single strands. Further, an elongation reaction is carried out under the conditions that permit carrying out an appropriate elongation by using the first primer 113a and the second primer 113b with the resultant single strand structure acting as a template, as shown in FIG. 11(B). Further, the nuclease enzyme that decomposes the nucleic acid from the 5'-terminal is subjected to a reaction under the conditions that permit obtaining an appropriate decomposing activity, as shown in FIG. 11(C). The nucleic acid not having a nuclease-resistant nucleotide contained in the exposed 5'-terminal, said nucleic acid being included in the nucleic acids present in the reaction system, is decomposed by the nuclease enzyme. As a result, it is possible to obtain the detection target structure 116 which comprises a protective strand 114a' and a protective strand 114b, as shown in FIG. 11(C).

It is possible to detect the target nucleotide sequence by allowing a nucleic acid probe 115 to be hybridized with the detection target structure 116 thus obtained and by detecting the particular hybridization as in the other embodiments described previously, as shown in FIG. 11(D).

In the present invention, it is possible to use nuclease such as exonuclease that decomposes a nucleic acid from the 5'-terminal as the nuclease enzyme. For example, T7 Gene6 Exonuclease can be used preferably as the nuclease enzyme.

The target nucleotide sequence 112 that can be used in the present embodiment is not particularly limited as long as the researcher wishes to detect the particular sequence. Also, it suffices for the target nucleotide sequence to have a length equal to the length of 5 bases to 300 bases, preferably equal to the length of 9 bases to 50 bases. Also, it is possible for the target nucleotide sequence 112 to be present in the vicinity of the 3'-terminal of the target nucleic acid. It is also possible for the target nucleotide sequence 112 to include the 3'-terminal as in the other embodiments described previously or to be positioned away from the 3'-terminal so as not to include the 3'-terminal.

The first primer 113a used in the present embodiment is made of a nucleic acid having a specified portion that is resistant to the nuclease enzyme and also having another portion corresponding to the target nucleotide sequence, which is not resistant to the nuclease enzyme. It is possible for the first primer 113a to be formed of any kind of a nucleic acid as long as the conditions given above are satisfied. The length of the first primer nucleic acid 113a should be sufficient for obtaining hybridization specific to the sequence of the first primer selected by the researcher. Also, the first primer nucleic acid 113a should have a length longer than the length of the target nucleotide sequence referred to above. For example, the first primer 113a used in this embodiment of the present invention should have a length equal to the length of at least 6 nucleotides, preferably equal to the length of at least 10 nucleotides. As described above, the nuclease enzyme-resistant portion of the first primer 113a used in this embodiment is that portion complementary to the nucleotide sequence of a part of the target nucleic acid 111a, the part being adjacent to the target nucleotide sequence 112 which is present in the vicinity of the 3'-terminal of the target nucleic acid 111a or on the 5'-terminal side relative to the target nucleotide sequence 112. It suffices for the length of nuclease-resistant portion of the first primer 111a to be 1 to 300 nucleotides, preferably 1 to 50 nucleotides, and more preferably 1 30 nucleotides. The portion of the first primer 113a which anneals on the target nucleotide sequence 112 is formed of a nucleotide that is not resistant to the nuclease enzyme. Also, it is possible for the first primer 113a to contain one or more nuclease-non-resistant nucleotides on the 3'-terminal side relative to the nuclease-resistant nucleotides.

It suffices for the second primer 113b used in this embodiment to contain at least one nuclease-resistant nucleotide on the 5'-terminal and to contain a nucleotide that is not resistant to the nuclease enzyme in another portion. It suffices for the total length of the second primer to be equal to the length of 5 to 100 nucleotides, preferably of 6 to 50 nucleotides, and more preferably of 10 to 35 nucleotides. Also, the nuclease-non-resistant portion in the second primer 113b, should be formed of about 4 to about 99 nucleotides, preferably about 5 to about 49 nucleotides, and more preferably about 9 to 34 nucleotides.

In this embodiment, the desired nucleotide may be rendered nuclease-resistant by modifying the nucleotide with use of a known means per se for rendering the nucleic acid resistant to the nuclease enzyme. The known means include, but not limited to, the phosphorothioate modification applied to the phosphodiester bond though the known means noted above.

The conditions under which the optimum elongation can be obtained, which is used herein, denote the conditions that, for example, an enzyme having a nucleotide elongation activity, a mono-nucleotide units incorporated into the elongated nucleic acid during the elongation stage, and a coenzyme required for the elongation reaction are present together, and that various conditions such as the temperature and the pH value can be controlled appropriately. The enzyme used in the present embodiment may be the similar enzyme that can be used in Embodiment 1 described previously. Also, the conditions such as an appropriate temperature control and the pH value control are similar to those described previously in conjunction with the first embodiment.

In this embodiment, an additional elongation is performed after step (A) in FIG. 11. It is also possible to carry out further additional elongation reactions repeatedly. To be more specific, in the method according to the tenth embodiment, it is possible to carry out an amplifying reaction which comprises twice or more of the elongation reaction. The particular amplification can be performed by any of known amplifying means. To be more specific, the amplifying means that can be employed in the present embodiment includes, for example, PCR amplification, nucleic acid strand amplification (NASBA), transcription mediated amplification (TMA), ligase strand reaction (LCR), strand displacement amplification (SDA), isothermal and chimeric primer-initiated amplification of nucleic acids (ICAN), a rolling circle amplification (RCA) method, and a loop mediated amplification (LAMP) method. In this embodiment, it is possible to manufacture a large number of detection target structures simultaneously by carrying out the amplifying reaction.

In the case of preparing a detection target structure according to the present invention in accordance with the tenth embodiment given above by using the target nucleic acid contained in the sample, it suffices for the sample to contain the target nucleic acid. However, it is not absolutely necessary for the sample to contain the target nucleic acid alone. In other words, it is possible for the sample to contain the target nucleic acid together with another nucleic acid. However, in order to carry out the reaction as desired in accordance with the embodiment of the present invention, it is preferred that the amount of the target nucleic acid contained in the sample to be not smaller than about 3%, preferably not smaller than 10%, and more preferably not smaller than 20%, based on the total amount of the nucleic acids contained in the sample. Also, it is possible to amplify, as required, the target nucleic acid by any kind of the known amplifying means before the target nucleic acid is subjected to the method according to the present invention. To be more specific, the amplifying means that can be employed in the present embodiment includes, for example, PCR amplification, nucleic acid strand amplification (NASBA), transcription mediated amplification (TMA), ligase strand reaction (LCR), strand displacement amplification (SDA), isothermal and chimeric primer-initiated amplification of nucleic acids (ICAN), a rolling circle amplification (RCA) method, and a loop mediated amplification (LAMP) method.

By the method according to the embodiment described above, it is possible to design the target nucleotide sequence freely in a smaller number of process steps. In addition, the detection target structure can be prepared easily in a short time. It follows that the economic advantage and the operability can be improved.

### (11) Eleventh embodiment:

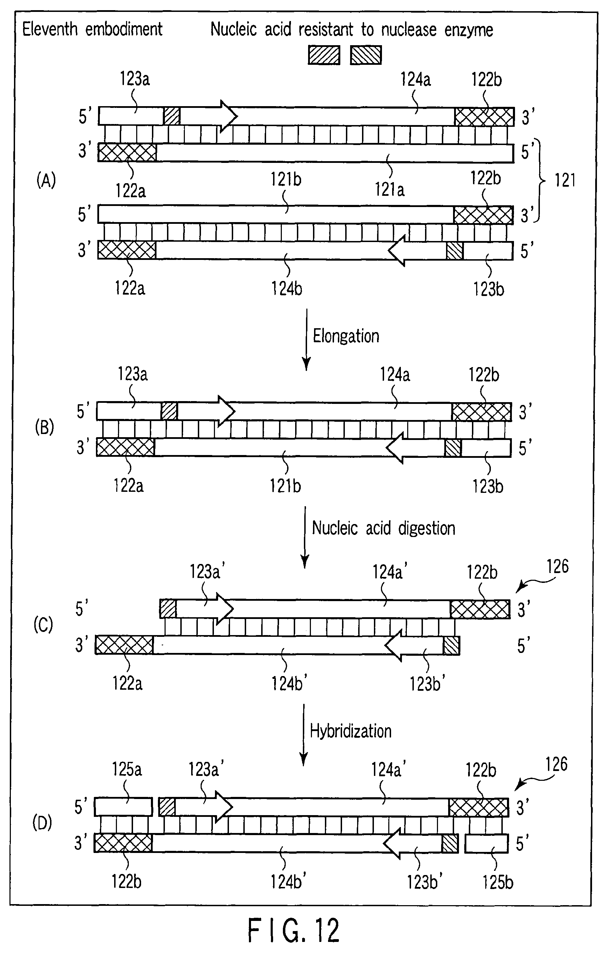

An eleventh embodiment of preparing a detection target structure according to the present invention will now be described with reference to FIG. 12. As shown in FIG. 12(A), a target nucleic acid 121 providing a basis for the manufacture of a detection target structure is formed of a double stranded target nucleic acid 121 comprising a first target nucleic acid 121a and a second target nucleic acid 121b. The eleventh embodiment covers the case where a target nucleotide sequences 122a and 122b are present in the vicinity of the 3'-terminals of the first and second target nucleic acids, respectively. In other words, the eleventh embodiment is a modification of the tenth embodiment.

An elongation reaction is carried out by using a first primer 123a having a sequence complementary to the first target nucleotide sequence 122a in the vicinity of the 3'-terminal of the first target nucleic acid 121a and a second primer 123b having a sequence complementary to the second target nucleotide sequence 112b, as shown in FIG. 12(A) so as to obtain a double stranded structure. Then, the double stranded structure thus obtained is denatured so as to obtain a single stranded structure. Further, the single stranded structure thus obtained is elongated again by using the first primer 123a, the second primer 123b and an enzyme having a nucleotide elongation activity, as shown in FIG. 12(B). Then, the nucleic acid that is not resistant to a nuclease enzyme is decomposed from the 5'-terminal by using a nuclease enzyme. As a result, obtained is a point-symmetric detection target structure 126 which includes target nucleotide sequences 122a, 122b that are present as a single stranded nucleic acid portion and a double strand nucleic acid portion consisting of complementary strands 124a' and 124b', as shown in FIG. 12(C).

It should be noted that each of the primers 123a and 123b contains a nucleotide that is not nuclease-resistant on the 5'-terminal side thereof. It suffices for the nuclease-non-resistant nucleotides to be present in the portions corresponding to the target nucleotide sequences 122a and 122b. Also, the nucleotide portion that is resistant to a nuclease enzyme is present in the sequence complementary to the region of the target nucleic acid which is on the 5'-terminal side relative to the position of the target nucleotide sequence.

In the eleventh embodiment, the nuclease-non-resistant nucleotide that is present on the 5'-terminal of each protective strand 124a and 124b, is decomposed by a nuclease enzyme after the elongation reaction. It follows that it is possible for the basic construction of each of the first primer 123a and the second primer 123b used in the eleventh embodiment to be substantially equal to that of the first primer described previously in conjunction with the tenth embodiment. To be more specific, a first portion of the primer having a sequence complementary to the target nucleotide sequence are made of the nuclease-non-resistant nucleotides, while a second portion of the primer positioned on the 3'-terminal side relative to the first portion comprises one or more nuclease resistant nucleotides. Preferably, the second portion of the primer is adjacent to the sequence of the first portion which is complementary to the target nucleotide sequence. The eleventh embodiment can be performed by the method similar to that of the tenth embodiment except that the constructions of the first primer 123a and the second primer 123b. Further, the eleventh embodiment can be modified as desired, in a similar manner as the modification of the tenth embodiment.

The target nucleotide sequence 112a, 112b can be detected by reacting the nucleic acid probes 125a and 125b simultaneously or sequentially with the detection target structure 126 thus obtained, followed by detecting the hybridization by the method similar to that employed in the other embodiments, as shown in FIG. 12(D).

By the method according to an embodiment of the present invention described above, it is possible to design the target nucleotide sequence freely in a smaller number of process steps. In addition, the detection target structure can be prepared easily in a short time. It follows that the economic advantage and the operability can be improved.

### 4. Detection target structure:

The detection target structure prepared by the method of the present invention described above is also included in the technical scope of the present invention. Also, the detection target structure according to the present invention can be used preferably for the detection of the target nucleotide sequence or the target nucleic acid containing the target nucleotide sequence. Also, the detection target structure prepared by the method of the present invention can be used under the state in which the structure is free in a solution, or under the state in which the structure is immobilized on the solid support such as beads or substrates. In other words, the present invention covers both the free detection target structure and the detection target structure immobilized on a solid support.

In general, the detection of the target nucleotide sequence is attained by hybridization with a nucleic acid probe complementary to the target nucleotide sequence, followed by detecting the hybridization. In many cases, the target nucleotide sequence is not present singly in a sample, but is present together with a nucleic acid having a plurality of different sequences or is contained in a target nucleic acid which comprises additional nucleotide sequences together with the target nucleotide sequence. Alternatively, a large number of target nucleotide sequences and target nucleic acids having the same sequence are present together in a manner to form a group. It follows that it is possible to bring about a secondary structure owing to the self-complementarity of the target nucleotide sequence or the target nucleic acid containing the target nucleotide sequence, as well as cross hybridization with another sequence. If such an unexpected hybridization is occurred, the detecting accuracy of the target nucleotide sequence is lowered. However, according to the detection target structure of the present invention, it is possible to suppress the generation of the self-complementarity and an unintentional hybridization caused by the complementarity with another sequence.

### 5. Detection method of target nucleotide sequence:

The method of detecting the target nucleotide sequence or the target nucleic acid by using the detection target structure prepared by the method of the present invention is also included in the technical scope of the present invention.

To be more specific, prepared is a sample that is to be detected. Then, a nucleic acid containing a target sequence is amplified as desired, followed by preparing a detection target structure by the method according to the present invention. In the next step, a nucleic acid probe complementary to the target nucleic acid that is to be detected is subjected to a reaction under the conditions that permit an appropriate hybridization relative to the prepared detection target structure. As a result, hybridization is occurred between the nucleic acid probe and the target nucleotide sequence in the case where the target nucleic acid is present in the sample. It follows that, if the coupling owing to the hybridization is detected, it is possible to determine the presence of the target nucleotide sequence or the target nucleic acid in the sample.

The known amplifying means can be used in the present invention for amplifying the nucleic acid. The amplifying means that can be employed in the present invention include, for example, PCR amplification, nucleic acid strand amplification (NASBA), transcription mediated amplification (TMA), ligase strand reaction (LCR), strand displacement amplification (SDA), isothermal and chimeric primer-initiated amplification of nucleic acids (ICAN), a rolling circle amplification (RCA) method, and a loop mediated amplification (LAMP) method. It is possible for those skilled in the art to select the appropriate conditions that permit achieving an appropriate hybridization in view of the various conditions such as the kind of the base contained in the target nucleotide sequence, the kind of the nucleic acid probe included in the nucleic acid probe-immobilized support, as well as the kind and state of the sample nucleic acid.

The general detection means, which is known and which is worked by using a nucleic acid probe, can be used in the detection method of the target nucleotide sequence using the detection target structure according to an embodiment of the present invention. The particular detecting means includes, for example, a detection method in which a fluorescent marker or other detectable markers such as a chemical luminescent label and a dye are imparted in advance to the nucleic acid probe and the marker is detected, a detection method that utilizes the detection of an intercalater which is capable of specifically binding to the hybridized portion, and an electrochemical detection method using a substance electrochemically recognizing the hybridized portion.

### 6. Assay kit:

### (1) Kit for preparing detection target structure:

A kit for preparing a detection target structure according to an embodiment of the present invention is also included within the technical scope of the present invention.

It suffices for the kit for preparing a detection target structure according to an embodiment of the present invention to comprise at least one of the various primers described above, a nucleic acid synthetase enzyme, mononucleotides for elongation reaction, a reagent required for obtaining a stopper nucleic acid, a nuclease enzyme, a reagent required for rendering the nucleic acid to be nuclease-resistant, a nucleic acid probe, a marker substance and other reagents for labeling the nucleic acid probe, and a desired solid support. Also, it is possible for the kit noted above to comprise some of these items in combination.

### (2) Assay kit for detecting target nucleotide sequence:

The present invention also provides an assay kit for detecting the target nucleotide sequence by using a detection target structure according to the embodiment of the present invention described above. It suffices for the assay kit for detecting the target nucleotide sequence to comprise a detection target structure according to the present invention, a nucleic acid primer, a detectable marker substance for detecting the hybridization, and at least one of various reagents used for the detection. Also, it is possible for the assay kit noted above to comprise some of these items in combination. Further, it is possible for the assay kit to comprise at least one item or a plurality of items used in the kit for preparing the detection target structure and selected from the group consisting of the various primers referred to above, a nucleic acid synthetase enzyme, mononucleotides for elongation reaction, a reagent required for obtaining a stopper nucleic acid, a nuclease enzyme, reagents required for rendering the nucleic acid to be nuclease-resistant, a nucleic acid probe, a marker substance and a reagent for labeling a nucleic acid probe, a solid support such as beads and a substrate, and a nucleic acid probe-immobilized solid support.

### [EXAMPLES]

### Example 1:

The fourth embodiment shown in FIG. 5, which uses a stopper nucleic acid, was carried out as Example 1 of the present invention, and the resultant experimental data were compared with the results obtained by the conventional method. Specifically, a detection target structure was prepared by each of the method of the present invention and the conventional method by utilizing, as a target nucleic acid, a single stranded DNA corresponding to a SNP-containing part of coding region of MBL gene. Then, SNP of the MBL gene was detected by using a nucleic acid probe-immobilized solid support for detection of SNP on the region noted above. The specific method was as follows.

A single stranded DNA having a sequence (i.e., sequence ID No. 1) equal to that of the region containing SNP to be detected in the MBL gene was used as a target nucleic acid, and the concentration of the target nucleic acid was controlled to have a final concentration of about 10¹² copies/mL.

Also, elongation of the protective strand was carried out by using a primer in the presence of a stopper nucleic acid. To be more specific, 3'-phosphorylated oligo DNA (sequence ID No. 2) having a final concentration of 0.4 *µ*M was used as a stopper nucleic acid, and oligo DNA (sequence ID No. 3) having a final concentration of 0.2 *µ*M was used as a primer. These stopper nucleic acid and primer were added to the target nucleic acid together with a heat-resistant DNA synthetase enzyme, i.e., Pyrobest DNA polymerase (Takara), having a final concentration of 0.04U/*µ*L. The sequences of the stopper nucleic acid and the primer were as follows:

Sequence ID No. 2:
GCCTAGACACCTGGGTTGCTGCTGGAAGACTATAAAC

Sequence ID No. 3:
CATGGTCCTCACCTTGGTGT

Then, a reaction was carried out at 66°C for 10 minutes under an optimum enzyme reaction solution composition. After the reaction, 1/9 volume of 20 x SSC buffer solution and 1/9 volume of 10 mM EDTA solution were added to the reaction mixture, and the resultant reaction mixture was added to a nucleic acid probe-immobilized chip prepared in advance. Under this condition, hybridization was carried out at 35°C for one hour. The nucleic acid probe-immobilized chip used in this stage contained, as a nucleic acid probe, a nucleic acid having a sequence ID No. 4 and a sequence ID No. 5, which was immobilized to a gold electrode included in the solid support by introducing a thiol group to the 3'-terminal. After the hybridization, washing with 0.2xSSC was performed for 15 minutes and, finally, the electric current response of Hoechst 33258 which was used as an intercalater was measured.

The conventional method providing a control case for comparison was performed as follows. Specifically, a single stranded DNA providing a target nucleic acid was mixed with a single stranded protective strand, i.e., a nucleic acid having a sequence ID No. 6, and the mixture was thermally denatured within a 2 × SSC buffer solution at 95°C for 5 minutes, followed by gradually cooling the mixture from 95°C to 30°C over 30 minutes for the annealing purpose. The detection target structure obtained by the conventional method after the annealing was added to a nucleic acid probe-immobilized chip similar to that referred to above, i.e., a chip having a nucleic acid having a sequence ID No. 4 and a sequence ID No. 5 immobilized as a nucleic acid probe on a gold electrode of a solid support. Then, hybridization was performed at 35°C for one hour. After the hybridization, washing with 0.2 × SSC was performed for 15 minutes and, finally, the electric current response of Hoechst 33258 was measured.

As another conventional method, a target nucleotide sequence was detected by using, as a detection target structure, a single stranded target nucleic acid (sequence ID No. 1) that did not contain a protective strand. A nucleic acid probe-immobilized chip similar to that described above, which had the sequence ID No. 4 and the sequence ID No. 5, was prepared by adding a nucleic acid probe, and the hybridization was performed similarly. After the hybridization, the washing with 0.2 × SSC was performed for 15 minutes and, finally, the electric current response of Hoechst 33258 was measured.

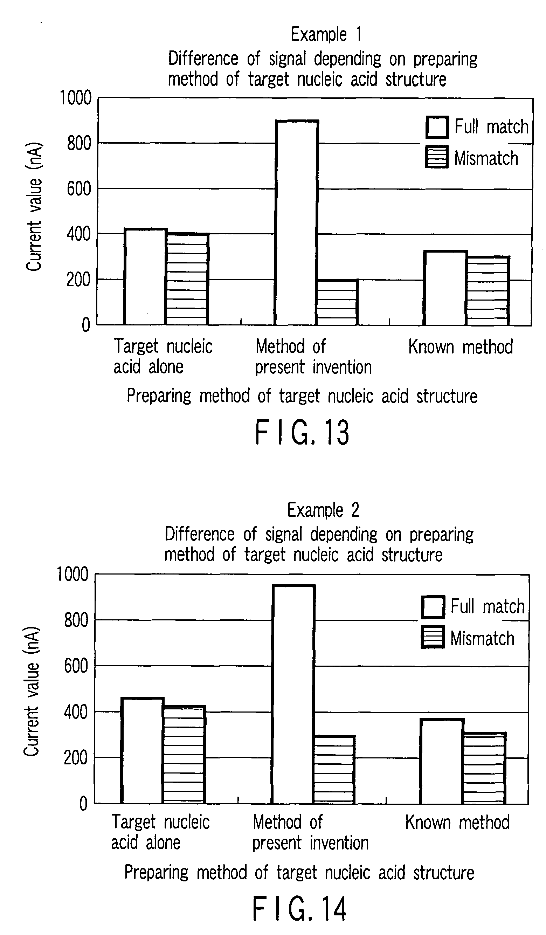

FIG. 13 is a graph showing the result. As a result of the hybridization described above, it was possible to detect a current signal indicating that a specific hybridization relative to the nucleic acid probe for detecting the corresponding SNP was generated only from the experimental section of the present invention described above, as shown in FIG. 13. Note that in the experimental section of the present invention, use was made of the detection target structure prepared by using 3'-phosphorylated oligo DNA having the sequence ID No. 2 as a stopper nucleic acid and oligo DNA having the sequence NO. 3 as a primer, and by adding a heat resistant DNA synthetase enzyme to a mixture of the stopper nucleic and the primer noted above.

Further, a comparative experiment was conducted by using a target nucleic acid having a different sequence. In any of the cases, a stable and specific signal was detected in only the case where the detection was performed by using a detection target structure prepared by the method of the present invention. Also, the conventional method covered the cases where the detection was performed by using a single stranded DNA consisting of a target nucleic acid and not accompanied by a protective strand as a detection target structure, and where the detection was performed by using a detection target structure prepared by mixing a target nucleic acid with a protective strand that was synthesized separately, followed by thermally denaturing and annealing the mixture. In any case of employing the conventional methods, it was impossible to obtain a desirable result as in the case shown in FIG. 13.

Also, a target nucleotide sequence was detected by preparing a detection target structure in accordance with the sixth example of the present invention shown in FIG. 7. To be more specific, a double strand nucleic acid was used as the target nucleic acid, and the target nucleotide sequences were provided by the different portions. Under this condition, the target nucleotide sequence was detected by preparing the detection target structure from each of the strands included in the double stranded target nucleic acid. As a result, it was possible to confirm the specific hybridization in only the case where the detection target structure was prepared by the method of the present invention.

As apparent from the experimental data given above, it has been found that it is possible for the method of the present invention to detect the target nucleic acid specifically, efficiently and with a higher sensitivity, compared with the conventional method. It follows that the present invention makes it possible to improve the economic advantage and the operability in preparing a target DNA.

### Example 2:

In Example 2, a desired detection target structure was prepared by controlling the annealing position of a primer as in the method of embodiment 1 of the present invention shown in FIG. 2, and the target nucleotide sequence was detected by using the detection target structure thus prepared. The result thus obtained was compared with the result obtained from the conventional method.

A solution containing an MxA gene was used as a sample, and a single stranded DNA (sequence ID No. 7) corresponding to the region including SNP of an MxA gene promoter was used a target nucleic acid. Also, the sequence including the SNP was used as a target nucleotide sequence. For each of these target nucleic acid and target nucleotide sequence, a detection target structure was prepared by each of the method of the present invention and the conventional method so as to detect the target nucleotide sequence. The target nucleotide sequence was detected by determining the presence or absence of hybridization relative to a nucleic acid probe-immobilized solid support obtained by immobilizing a nucleic acid probe having a sequence complementary to the target nucleotide sequence on the support. By this detection, it is possible to detect the gene type of SNP included in the MxA gene promoter.

A target single stranded DNA having a same sequence as the region containing SNP which is to be detected in the MxA gene was prepared so that the target nucleic acid was controlled to have a final concentration of about 10¹² copies/ml. In addition, an oligo DNA (sequence ID No. 8) was prepared as a primer. The primer having a final concentration of 0.4 *µ*M and an enzyme for PCR, i.e., Pyrobest DNA polymerase (Takara), having a final concentration of 0.04U/µl were added to the target nucleic acid. The sequence of the primer was as follows:

Sequence ID No. 8:

GCCGCCCTCAGCACAGGGTCTGTGAGTTTCATTTC

The elongation reaction was carried out at 66°C for 10 minutes under an optimum enzyme reaction solution composition. After the reaction, 1/9 volume of 20 × SSC buffer solution and 1/9 volume of 10 mM EDTA solution were added to the reaction mixture, and the resultant mixture was added to a nucleic acid probe-immobilized chip prepared in advance so as to carry out the hybridization at 35°C for one hour. In the nucleic acid probe-immobilized chip, the probes each of which has the sequence identified by sequence ID No. 9 or the sequence ID No. were immobilized on a gold electrode by introducing a thiol group into the 5'-terminal. After the hybridization, the washing with 0.2 × SSC was carried out for 15 minutes and, finally, the electric current response of Hoechst 33258 was measured.

The conventional method providing a comparative case was carried out as follows. Specifically, a single stranded DNA providing a target nucleic acid was mixed with a single stranded protective strand, i.e., a nucleic acid having the sequence ID No. 11, which was synthesized separately, followed by gradually cooling the mixture within a 2 × SSC buffer solution from 95°C to 30°C over 30 minutes so as to achieve an annealing. The detection target structure according to the conventional method, which was obtained after the annealing described above, was added to tow nucleic acid probe-immobilized chips similar to that described previously, i.e., each chip prepared by immobilizing nucleic acids including the sequence ID No. 9 or the sequence ID No. 10 as a nucleic acid probe on a gold electrode of a solid support. Then, hybridization was carried out at 35°C for one hour. After the hybridization, the washing with 0.2 × SSC was performed for 15 minutes and, finally, the electric current response of Hoechst 33258 was measured.

As an additional conventional method, the target nucleotide sequence was detected by using a single stranded target nucleic acid (sequence ID No. 7) that did not include a protective strand as a detection target structure. More specifically, the detection target structure was added to a nucleic acid probe-immobilized chip similar to that described above in which two nucleic acid probes each containing the sequence identified ID No. 9 or the sequence ID No. 10 was immobilized, and then, hybridization was carried out in a similar manner as described previously so as to measure the electric current response.

As a result, as shown in FIG. 14, from only the experimental section in which use was made of the detection target structure prepared by the method of the present invention, an electric current signal was detected which signal indicates that the hybridization specific to the nucleic acid probe capable of detecting the corresponding SNP was performed. Further, a comparative experiment was conducted similarly in respect of a plurality of target nucleic acid having different kinds of sequences. In any case of the comparative experiment, a stable and specific signal was detected from only the sample prepared by the method based on the present invention. On the other hand, the conventional method covered the case where the detection was performed by using a detection target structure which includes a single stranded DNA comprising a target nucleic acid and not includes a protective strand, and the case where the detection was performed by using a detection target structure prepared by mixing a target nucleic acid with a protective strand that was synthesized separately, followed by thermally denaturing and annealing the mixture. In almost all the cases of employing the conventional method, it was impossible to obtain a desirable result as in the cases shown in FIG. 14.

In addition, a detection target structure was prepared in accordance with the third embodiment of the present invention shown in FIG. 4 so as to detect the target nucleotide sequence. To be more specific, a double stranded nucleic acid was used as a target nucleic acid, and the portions thereof differing from each other are used as the target nucleotide sequences. Under this condition, a detection target structure was prepared from each strand included in the double stranded target nucleic acid. Further, a detection target structure was prepared in accordance with the seventh embodiment of the present invention shown in FIG. 8 so as to detect the target nucleotide sequence. To be more specific, a double stranded nucleic acid was used as a target nucleic acid, and the portions thereof complementary to each other was used as the target nucleotide sequences. Under this condition, a detection target structure was prepared from each strand included in the double stranded target nucleic acid. To reiterate, two kinds of the detection target structures were prepared from a double stranded target nucleic acid in accordance with the method of the present invention, and the target nucleotide sequence was detected by using the detection target structures thus prepared. In each of these cases, it was possible to confirm the hybridization specific to the target nucleotide sequence. The comparison between the particular result and the result of the conventional method clearly indicates that the method of the present invention permits detection of the target nucleotide sequence with sensitivity higher than that for any of the conventional methods.

As apparent from the experimental data described above, the method of the present invention makes it possible to detect the target nucleic acid specifically, efficiently and with a high sensitivity, compared with the conventional method. It follows that it is possible to improve the economic advantage and the operability in preparing a target DNA.

### Example 3:

Various conditions were examined in respect of the method of preparing the detection target structure according to the present invention, which uses the stopper nucleic acid described above. A target nucleic acid structure was prepared by using, as a target nucleic acid, a single stranded DNA corresponding to the region containing SNP in the coding region of MBL gene, and by changing the kind of the oligo DNA used as a primer. Then, SNP of the MBL gene was detected by using a nucleic acid probe-immobilized solid support for detecting SNP on the same region.

A target nucleic acid prepared in this Example was a single stranded DNA having a sequence (sequence ID No. 1) equal to that of the region containing SNP to be detected in the MBL gene. The target nucleic acid was synthesized and amplified so as to have a final concentration of about 10¹² copies/ml. As a stopper nucleic acid, prepared was 3'-phosphorylated oligo DNA, which is represented by the sequence ID No. 2, the sequence ID No. 12, the sequence ID No. 13, and the sequence ID No. 14, which are given below. Then, added to the target nucleic acid were any one of the stopper nucleic acids having a final concentration of 0.4 *µ*M, i.e., 3'-phosphorylated oligo DNA, which is represented by the sequence ID No. 2, the sequence ID No. 12, the sequence ID No. 13, and the sequence ID No. 14, in addition to a primer oligo DNA (sequence ID No. 3) having a final concentration of 0.2 *µ*M, and a heat resistant DNA synthetase enzyme having a final concentration of 0.04U/*µ*l, i.e., Pyrobest DNA polymerase (Takara). The sequences of the stopper nucleic acids and the primer used were as follows:

Sequence ID No. 2:
GCCTAGACACCTGGCGTTGCTGCTGGAAGACTATAAAC

Sequence ID No. 12:
GCCTAGACACCTGGCGTTGCTGCTGGAAGACTATAAA

Sequence ID No. 13:

CACCTGGCGTTGCTGCTGGAAGACTATAAA

Sequence ID No. 14:
CCTGGCGTTGCTGCTGGAAGACTATAAAC

Sequence ID No. 3:
CATGGTCCTCACCTTGGTGT

Then, the nucleic acid was elongated by carrying out a reaction at 66°C for 10 minutes under the optimum enzyme reaction solution composition. After the reaction, 1/9 volume of 20 × SSC buffer solution and 1/9 volume of 10 mM EDTA solution were added to the reaction mixture, followed by performing the hybridization at 35°C for one hour with two nucleic acid probes which have a sequences represented by the sequence ID No. 4 and the sequence ID No. 5, respectively. Note that the probes had been immobilized on a gold electrode in advance, by introducing a thiol functionality into the 3'-terminal thereof. After the hybridization, washing with 0.2 x SSC was performed for 15 minutes and, finally, the electric current response of Hoechst 33258 was measured.

As a result, a specific signal was obtained from any of the experimental sections. However, in the experimental section in which the sequence ID No. 2 or the sequence ID No. 3 was used as the 3'-phosphorylated oligo DNA acting as a stopper nucleic acid and in which the oligo DNA of sequence ID No. 3 acting as a primer and a heat resistant DNA synthetase enzyme were added, a current signal indicating the hybridization specific to the nucleic acid probe for detecting the corresponding SNP was detected with higher sensitivity, compared with the case where the sequence ID No. 13 or the sequence ID No. 14 was used as the 3'-phosphorylated oligo DNA acting as a stopper nucleic acid. The experimental data clearly indicates that, in preparing a target DNA by the method of the present invention, it is more desirable to design that the 3'-terminal of the 3'-phosphorylated oligo DNA acting as a stopper nucleic acid and the 5'-terminal of the nucleic acid probe are positioned adjacent to each other.

### Example 4:

In respect of the method of preparing a desired detection target structure according to the present invention by controlling the annealing position of the primer, conditions for preparing the detection target structure were further studied. A single stranded DNA corresponding to the region containing SNP of the MxA gene promoter region was used as a target nucleic acid. A plurality of sequences of the primer used was prepared, and a detection target structure was created by using the plural sequences of the primer thus prepared. The detection target structure thus obtained was added to a nucleic acid probe-immobilized solid support for detecting SNP on the region so as to carry out a reaction, thereby detecting the SNP of the MxA gene.

A single stranded DNA (sequence ID No. 7) having a sequence equal to that of the region containing SNP to be detected in the M×A gene was used as a target nucleic acid. The particular target nucleic acid was synthesized nad amplified. Prepared as the primer was a nucleic acid having any of three kinds of the sequences (i.e., the sequence ID No. 8, the sequence ID No. 15, and the sequence ID No. 16). The primer having a final concentration of 0.4 *µ*M and an enzyme for PCR having a final concentration of 0.04U/*µ*l, i.e., Pyrobest DNA polymerase (Takara), were added to the target nucleic acid. The sequences of the added primer were as follows:

Sequence ID No. 8:
GCCGCCCTCAGCACAGGGTCTGTGAGTTTCATTTC

Sequence ID No. 15:

CCGCCCTCAGCACACAGGTCTGTGAGTTTCATTTC

Sequence ID No. 16:
CGCCCTCAGCACAGGGTCTGTGAGTTTCATTTC

A mixture of the target nucleic acid, the primer, and the enzyme was subjected to a reaction at 66°C for 10 minutes under the optimum enzyme reaction solution composition. After the reaction, 1/9 volume of 20 × SSC buffer solution and 1/9 volume of 10 mM EDTA solution were added to the reaction mixture, and the resultant mixture was added to a nucleic acid probe-immobilized chip prepared in advance so as to carry out the hybridization at 35°C for one hour. The nucleic acid probe-immobilized chip contained, as the nucleic acid probes, nucleic acids which had sequences represented by the sequence ID No. 9 and the sequence ID No. 5, respectively, and were immobilized on a gold electrode included in the support by introducing a thiol group into the 5'-terminal thereof. After the hybridization, washing with 0.2 × SSC was performed for 15 minutes and, finally, the electric current response of the Hoechst 33258 was measured.

As a result, detected was a current signal indicating that the hybridization specific to the nucleic acid probe for detecting the corresponding SNP was detected from any of the experimental sections. Among others, it was interesting to note that a difference in the obtained signal intensity was recognized depending on the sequence of the primer used. The relationship between the signal intensity and the primer sequence was in the order of the sequence ID No. 8 > the sequence ID No. 15 > the sequence ID No. 16. In other words, the experimental data clearly indicates that, in preparing a detection target structure by the method of the present invention, it is more desirable to design that the 5'-terminal of the primer used and the 3'-terminal of the nucleic acid probe are positioned adjacent to each other.

### Example 5:

A detection target structure was prepared by the method according to the fifth example of the present invention shown in FIG. 6, and the target nucleotide sequence was detected. In this Example, studied were the conditions in the case of using a double strand nucleic acid as a target nucleic acid. To be more specific, a PCR product corresponding to the SNP-containing region of the coding region in the MBL gene was used as a target nucleic acid, and a detection target structure according to the present invention was prepared under several different conditions. The SNP of the MBL gene was detected by using the detection target structure thus obtained and a nucleic acid probe-immobilized chip, the chip having a nucleic acid probe immobilized thereon for detecting the SNP on that region.

The PCR method was performed by using two primer oligo DNAs containing the sequence ID No. 17 and the sequence ID No. 3, respectively, and using the human genomic DNA of known SNP type as a template. By the PCR reaction, the region containing the SNP to be detected in the MBL gene was amplified. Added to the amplified product were 3'-phosphorylated oligo DNA (sequence ID No. 2), which was used as a stopper nucleic acid having a final concentration of 0.4 *µ*M, a heat resistant DNA synthetase enzyme having a final concentration of 0.04U/*µ*l, i.e., Pyrobest DNA polymerase (Takara), an oligo DNA (sequence ID No. 17) having a final concentration of 0.2 *µ*M, which was used as a first primer, and/or an oligo DNA (sequence ID No. 3) having a final concentration of 0.2*µ*M, which was used as a second primer. The sequences of the oligo DNA used were as follows:

Sequence ID No. 17:
GTCCCATTTGTTCTCACTGC

Sequence ID No. 3:
CATGGTCCTCACCTTGGTGT

Sequence ID No. 2:
GCCTAGACACCTGGCGTTGCTGCTGGAAGACTATAAAC

Then, the mixture was subjected to a reaction at 95°C for 5 minutes and at 66°C for 10 minutes under the optimum enzyme reaction solution composition. After the reaction, 1/9 volume of 20 × SSC buffer solution and 1/9 volume of 10 mM EDTA solution were added to the reaction mixture, and the resultant mixture was added to a nucleic acid probe-immobilized chip prepared in advance so as to carry out the hybridization at 35°C for one hour. The chip contained, as the nucleic acid probes, nucleic acids represented by the sequence ID No. 4 and the sequence ID No. 5 and immobilized on a gold electrode included in the chip by introducing a thiol group into the 3'-terminal thereof. After the hybridization, washing with 0.2 × SSC was performed for 15 minutes and, finally, the electric current response of the Hoechst 33258 was measured.

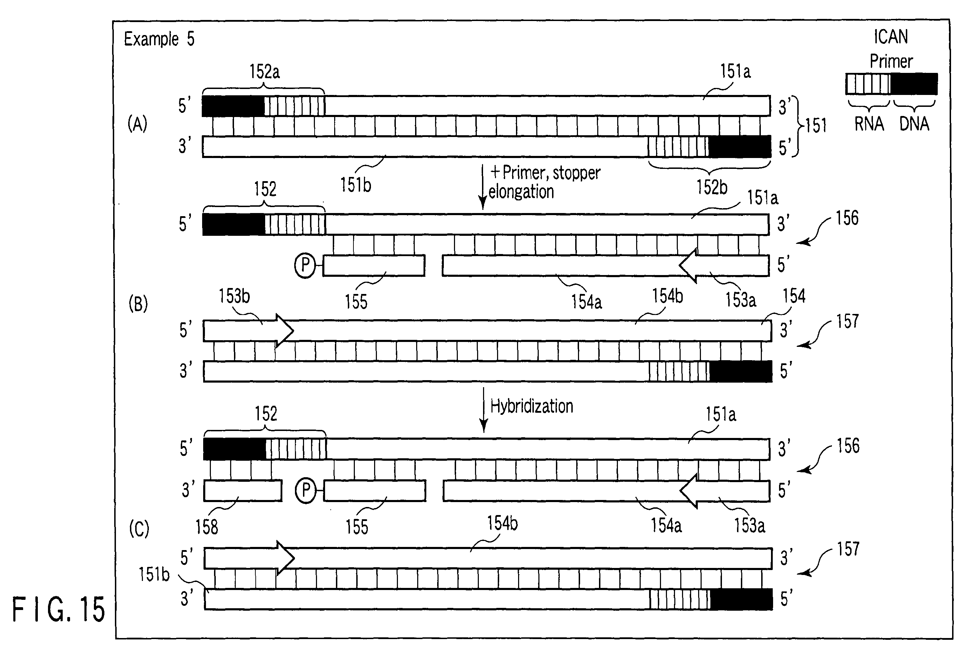

As a result, a signal indicating that the hybridization specific to the nucleic acid probe which serves to detect the corresponding SNP was detected from only the experimental section in which all of the oligo DNA having the sequence ID No. 17 and the oligo DNA having the sequence ID No. 3, which were used as the primers, the 3'-phosphorylated oligo DNA having the sequence ID No. 2, which was used as a stopper nucleic acid, and the heat resistant DNA synthetase enzyme were added to the PCR product. Further, a plurality of different kinds of additional target nucleotide sequences was prepared, and a similar experiment was conducted for these target nucleic acids. As a result, it has been found that a stable and specific signal was detected in only the case where the detection target structure was prepared by a method similar to that described above. On the other hand, it was impossible to obtain a desirable result in almost all the cases where the detection target structure was prepared by other methods. Also, when the amplified product was used as the target nucleic acid as described above, it was possible to use the amplified product obtained by any amplifying means other than the PCR method as the target nucleic acid. For example, as apparent from FIG. 15, even in the ICAN product, a satisfactorily specific detection of a target nucleotide sequence was confirmed in the case of using a detection target structure prepared by a method similar to that described above. The experimental data given above clearly support that, in the case of preparing a detection target structure according to the present invention by using a double stranded nucleic acid as a target nucleic acid, it is desirable for any strand complementary to the target nucleic acid not to be present in the hybridization solution as a free single stranded nucleic acid during the reaction with the nucleic acid probe. It follows that it is desirable to add the primer such that each of both strands of the double stranded target nucleic acid is allowed to form a double strand during the hybridization.

### Example 6:

In this Example, a detection target structure was prepared by the method according to the second embodiment of the present invention shown in FIG. 3 so as to detect the target nucleotide sequence. In this Example, studied were the conditions in the case of using a double strand nucleic acid as the target nucleic acid. To be more specific, a detection target structure according to the present invention was prepared under several different conditions by using a PCR product corresponding to the region containing the SNP of the M×A gene promoter as the target nucleic acid. The SNP of the MxA gene was detected by using the detection target structure thus obtained, and using a nucleic acid probe-immobilized chip which has the nucleic acid probe for detecting the SNP immobilized thereon.

The PCR method was performed by using two primer oligo DNAs containing the sequence ID No. 18 and the sequence ID No. 19, respectively, as a primer, and using the human genomic DNA of known SNP type as a template. The region containing the SNP to be detected in the MxA gene was amplified by the PCR method. Added to the amplified product were an oligo DNA (sequence ID No. 18) used as a first primer and/or an oligo DNA (sequence ID No. 19) used as a second primer having a final concentration of 0.4 /*µ*M, and an enzyme for PCR having a final concentration of 0.04U/*µ*l, i.e., Pyrobest DNA polymerase (Takara). The sequences of the oligo DNA used were as follows:

Sequence ID No. 18:
GAGCTAGGTTTCGTTTCTGC

Sequence ID No. 19:
CGCTAGTCTCCGCCACGAAC

Sequence ID No. 8:
GCCGCCCTCAGCACAGGGTCTGTGAGTTTCATTTC

Then, the mixture was subjected to a reaction at 95°C for 5 minutes and at 66°C for 10 minutes under the optimum enzyme reaction solution composition. After the reaction, 1/9 volume of 20 × SSC buffer solution and 1/9 volume of 10 mM EDTA solution were added to the reaction mixture, and the resultant mixture was added to a nucleic acid probe-immobilized chip prepared in advance so as to carry out the hybridization at 35°C for one hour. The nucleic acid probe-immobilized chip used here contained, as the nucleic acid probes, nucleic acids represented by the sequence ID No. 9 and the sequence ID No. 10, respectively, and immobilized on a gold electrode included in the chip by introducing a thiol group into the 5'-terminal thereof. After the hybridization, washing with 0.2 × SSC was performed for 15 minutes and, finally, the electric current response of the Hoechst 33258 was measured.

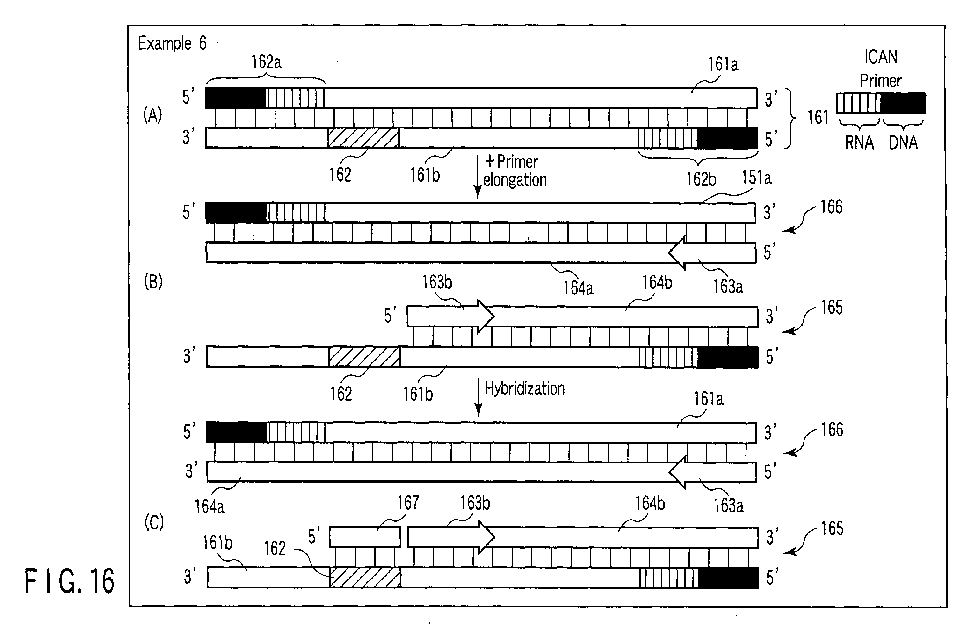

As a result, a signal indicating that the hybridization specific to the nucleic acid probe serving to detect the corresponding SNP was detected from only the experimental section in which all of the oligo DNA having the sequence ID No. 19, which was used as a first primer, the oligo DNA having the sequence ID No. 8, which was used as a second primer, and the heat resistant DNA synthetase enzyme were added to the PCR product. Further, a plurality of different kinds of target nucleic acids was prepared, and a similar experiment was conducted for these target nucleic acids. As a result, it has been found that a stable and specific current signal was detected in only the case where the detection target structure was prepared by a method similar to that described above. On the other hand, it was impossible to obtain a desirable result in almost all the cases where the detection target structure was prepared by other methods. Also, when the amplified product is used as the target nucleic acid as described above, it is possible to use, as the target nucleic acid, the amplified product obtained by any amplifying means other than the PCR method. For example, as apparent from FIG. 16, even in the ICAN product, a satisfactorily specific detection of a target nucleotide sequence was confirmed in the case of using a detection target structure prepared by a method similar to that described above. The experimental data given above clearly support that, in the case of preparing a detection target structure according to the present invention by using a double strand nucleic acid sample as a target nucleic acid, it is desirable for any nucleic acid complementary to the target nucleic acid not to be present in the hybridization solution as a free single stranded nucleic acid during the reaction with the nucleic acid probe. It follows that it is desirable to add the primers such that each of both strands of the double stranded target nucleic acid is allowed to form a double strand during the hybridization.

### Example 7:

In this Example, a detection target structure was prepared in accordance with the method of forming a single stranded nucleic acid portion by using a limited enzyme digestion as in the tenth embodiment of the present invention shown in FIG. 11 and the eleventh embodiment of the present invention shown in FIG. 12. The result of detecting the target nucleotide sequence by using the detection target structure thus prepared was compared with the result of detecting the target nucleotide sequence by using a detection target structure prepared by the conventional method. In this example, a rice genome was used as the target nucleic acid, and the judgment was performed as to whether or not PCR amplified fractions of a rice genome containing the target nucleotide sequence was present, by using a solid support having a nucleic acid probe immobilized thereon.

In the first step, the rice genomic DNA was extracted from polished rice and adjusted. The rice genomic DNA was amplified by the PCR by using four primers given below in respect of the region sandwiched by these four primers. The sequences of the primers used were as follows:

Sequence ID No. 20:  tacCTGGTTGATGTATACAGATCTGGTT

Sequence ID No. 21:  ATCCCTCGATCCCTCtagCATTAT

Sequence ID No. 22:  TACCTGGTTGATGTAtacAGATCTGGTT

Sequence ID No. 23:  atcCCTCGATCCCTCTAGCATTAT

The base represented by small letters in each of these sequence ID No. 20, sequence ID No. 21, sequence ID No. 22 and sequence ID No. 23 denotes that the base has a phosphorothioate modification applied to the phosphodiester bond present on the 3'-terminal side thereof.

The complete digestion was performed under the optimum conditions by adding T7 Gene6 Exonuclease and the attached buffer to the amplified product. After the reaction, 1/9 volume of 20 × SSC buffer solution and 1/9 volume of 10 mM EDTA solution were added to the enzyme reaction mixture, and the resultant mixture was added to a nucleic acid probe-immobilized chip prepared in advance so as to carry out the hybridization at 35°C for one hour. The nucleic acid probe-immobilized chip contained, as the nucleic acid probes, nucleic acids represented by the sequence ID No. 24, the sequence ID No. 25 and the sequence ID No. 26, the probes being immobilized on a gold electrode included in the chip by introducing a thiol group into the 5'-terminal thereof.

Nucleic acid probe:

Sequence ID No. 24: TACCTGGTTGATGTA

Sequence ID No. 25:  ATCCCTCGATCCCTC

Sequence ID No. 26: ACAACGCCTCCGATG (control

case)

In carrying out the hybridization reaction, experiments for the control cases were also conducted. To be more specific, the hybridization was similarly performed in respect of the detection target structure prepared without applying the T7 Gene6 Exonuclease treatment, the detection target structure in which the PCR amplification was performed by using primers to which the phosphorothioate modification was not applied, the detection target structure subjected to the T7 Gene6 Exonuclease treatment, and the detection target structure prepared in accordance with the conventional method disclosed in Japanese Patent Disclosure No. 11-332566 directed to a DNA detection method utilizing a partially double stranded DNA. After the hybridization, washing with 0.2xSSC was performed for 15 minutes and, finally, the electric current response of Hoechst 33258 was measured.

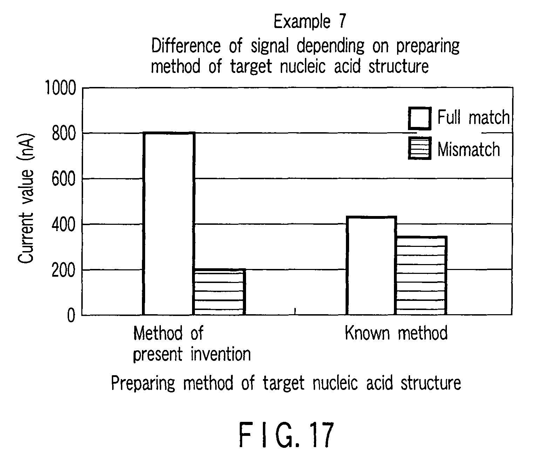

As a result, the experimental data indicating that the hybridization was generated was not obtained in any of the cases where the T7 Gene6 Exonuclease treatment was not applied to the PCR product, where the T7 Gene6 Exonuclease treatment was applied after PCR-amplification by using a primer that was not modified with phosphorothioate, and where the T7 Gene6 Exonuclease treatment was applied after performing the PCR by using the combination of the primers of sequence ID No. 20 and the sequence ID No. 23. Incidentally, in the detection target prepared in accordance with the DNA detection method utilizing the partially double stranded DNA, which is disclosed in Japanese Patent Disclosure No. 11-332566 referred to above, it was certainly possible to obtain a certain degree of signals. However, the background signal was increased and, thus, the S/N ratio was about 1.2. On the other hand, where the PCR was performed by the combination of the primers of sequence ID No. 20 and the sequence ID No. 21, the nucleic acid probe sequence ID No. 24 made it possible to obtain a specific signal with a large S/N ratio, which was much larger than 2, i.e., S/N >> 2, as shown in FIG. 17. Likewise, where the PCR was performed by the combination of primers of sequence ID No. 22 and the sequence ID No. 23, the nucleic acid probe of sequence ID No. 25 made it possible to obtain a specific signal with a large S/N ratio, which was much larger than 2, i.e., S/N >> 2, as shown in FIG. 17. Also, in the case where the PCR was performed by the combination of the primers of sequence ID No. 21 and the sequence ID No. 22, it was confirmed that the specific hybridization with both of the nucleic acid probes of the sequence ID No. 24 and the sequence ID No. 25 was performed with a high S/N ratio of S/N >> 2. Further, an experiment was conducted in respect to a multiplex PCR product in which a plurality of PCR fractions was present simultaneously. As a result, a high specificity was observed in the case where the detection target structure was prepared by the method of the present invention, compared with the case where the detection target structure was prepared by the conventional method. The experimental data given above indicates that, where the detection target structure is prepared by the method of the present invention, the economic advantage and the operability are improved, compared with the case where the detection target structure is prepared by the conventional method, so as to make it possible to detect the target nucleic acid specifically, efficiently and/or with a higher sensitivity.

### Example 8:

In this Example, the preparing method of the detection target structure according to the present invention was applied to the amplified product by the multiplex PCR, and the resultant product was used for the detection by the hybridization.

In order to amplify the region containing the SNP, which was selected from the group consisting of the MxA gene promoter region and the MBL gene code region, with the human genomic DNA of known SNP type used as a template, the multiplex PCR was performed by using the primers of sequence ID No. 17, the sequence ID No. 3, the sequence ID No. 18 and the sequence ID No. 19. After the reaction, added to the PCR product were a primer (sequence ID No. 8) having a final concentration of 0.4 *µ*M, 3'-phosphorylated oligo DNA (sequence ID No. 2) having a final concentration of 0.4 *µ*M and used as a stopper nucleic acid, two kinds of primers each having the sequence ID No. 17 or the sequence ID No. 19 and having a final concentration of 0.2 *µ*M, and an PCR enzyme having a final concentration of 0.04U/*µ*l, i.e., Pyrobest DNA polymerase (Takara), so as to carry out the reaction at 95°C for 5 minutes and at 66°C for 10 minutes.

The sequences of the oligo DNA used were as follows:

Sequence ID No. 17:
GTCCCATTTGTTCTCACTGC

Sequence ID No. 3:
CATGGTCCTCACCTTGGTGT

Sequence ID No. 18:
GAGCTAGGTTTCGTTTCTGC

Sequence ID No. 19:
CGCTAGTCTCCGCCACGAAC

Sequence ID No. 2:
GCCTAGACACCTGGCGTTGCTGCTGGAAGACTATAAAC

Sequence ID No. 8:
GCCGCCCTCAGCACAGGGTCTGTGAGTTTCATTTC

After the reaction, 1/9 volume of 20 × SSC buffer solution and 1/9 volume of 10 mM EDTA solution were added to the reaction mixture, and the resultant mixture was added to a nucleic acid probe-immobilized chip prepared in advance so as to carry out the hybridization at 35°C for one hour. The nucleic acid probe-immobilized chip used in this stage comprised, as the nucleic acid probes, nucleic acids represented by the sequence ID No. 4 and the sequence ID No. 5 and immobilized on a gold electrode included in the cip by introducing a thiol group into the 3'-terminal and additional nucleic acids represented by the sequence ID No. 9 and the sequence ID No. 10 and immobilized on a gold electrode included in the chi by introducing a thiol group into the 5'-terminal. After the hybridization, washing with 0.2 x SSC was performed for 15 minutes and, finally, the electric current response of the Hoechst 33258 was measured. As a result, a specific current signal indicating that the hybridization was occurred in the nucleic acid probe serving to detect the corresponding SNP.

As a result, it has been clarified that, in the case of preparing a detection target structure by the method according to the present invention and using the detection target structure thus prepared for detecting the target nucleotide sequence, it is possible to detect the target nucleotide sequence with a high specificity even in the case where a mixed solution containing a plurality of different kinds of DNA fractions is used a sample.

### Example 9:

In this Example, as in the eighth embodiment of the present invention shown in FIG. 9, prepared was a detection target structure that permits simultaneous detection of target nucleotide sequences at two portions on the single stranded target nucleic acid, and the target nucleotide sequences were detected by employing the hybridization using the detection target structure thus prepared.

Use was made of a target single stranded DNA as the target nucleic acid having a sequence (sequence ID No. 27) of the MBL gene, and having the SNP region positioned on both terminals thereof. The target nucleic acid was synthesized and amplified and, then, adjusted to have a final concentration of about 10¹² copies/ml. Then, a primer of sequence ID No. 28 having a final concentration of 0.4 *µ*M, 3'-phosphorylated oligo DNA of sequence ID No. 2 (a stopper nucleic acid) having a final concentration of 0.4 *µ*M, and an PCR enzyme having a final concentration of 0.04 U/*µ*l, i.e., Pyrobest DNA polymerase (Takara) were added to the target nucleic acid so as to carry out a reaction at 66°C for 10 minutes. The sequences of the oligo DNA used were as follows:

Sequence ID No. 2:

GCCTAGACACCTGGCGTTGCTGCTGGAAGACTATAAAC

Sequence ID No. 28:
CCCATCTTTGCCTGGGAAGCCGTTGATGCC

After the reaction, 1/9 volume of 20 × SSC buffer solution and 1/9 volume of 10 mM EDTA solution were added to the enzyme reaction mixture. Then, the resultant mixture was added to a nucleic acid probe having the sequence ID No. 4 and a nucleic acid probe having the sequence ID No. 5, as well as a nucleic acid probe having the sequence ID No. 29 and a nucleic acid probe having the sequence ID No. 30 so as to carry out the hybridization at 35°C for one hour. Note that each of the nucleic acid probes was immobilized in advance on a gold electrode of a chip by introducing a thiol group into the 5'-terminal thereof. After the hybridization, washing with 0.2×SSC was performed for 15 minutes and, finally, the electric current response of the Hoechst 33258 was measured.

As a result, a specific signal was detected, indicating that the hybridization was occurred in the nucleic acid probe serving to detect the corresponding SNP. In addition, a detection target structure in which target nucleotide sequences are present in at least three portions on a single stranded target nucleic acid was also prepared, on the basis of the similar design by the method according to the present invention such as the ninth embodiment shown in FIG. 10. The detection target structure having four target nucleotide sequence was prepared as described above, and these target nucleotide sequences at the four portions was detected by using the detection target structure thus prepared. As a result, it was confirmed that a specific hybridization was occurred.

The experimental data clearly supports that the target nucleotide sequences at a plurality of portions present on a single stranded target nucleic acid can be detected simultaneous by the method according to the present invention.

It should be noted that the detection target structure in each of Example 1 to Example 9 given above was detected by means of the electrochemical method utilizing a current response on a gold electrode. However, the detection target structure can also be detected in any of Example 1 to Example 9, by imparting a fluorescent marker to the prepared detection target structure by the known method, and by measuring the fluorescent intensity after reaction with the chip or the support having the corresponding nucleic acid probe immobilized thereon.

As described above in detail, the method based on the present invention makes it possible to obtain a degree of freedom in designing the target nucleic acid, to improve the specificity, efficiency, and the sensitivity in the detection of the target nucleic acid, and to manufacture a target nucleic acid structure with a low production cost and/or in a short time.

## Claims

1. A method of preparing a detection target structure useful for detecting the target nucleotide sequence, comprising:
reacting a target nucleic acid which contains a target nucleotide sequence positioned in the vicinity of 3'-terminal thereof, under the conditions that permit an appropriate elongation reaction, with;
a primer complementary to the sequence of a region of the target nucleic acid, said region being positioned on the 5'-terminal side relative to the 5'-terminal of the target nucleotide sequence, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

2. A method of preparing a detection target structure useful for detecting the target nucleotide sequence, comprising:
reacting a target nucleic acid which contains a target nucleotide sequence positioned in the vicinity of 5'-terminal thereof, under the conditions that permit an appropriate elongation reaction, with;
a stopper nucleic acid complementary to the sequence of a region of the target nucleic acid, said region being positioned on the 3'-terminal side relative to the 3'-terminal of the target nucleotide sequence, and said stopper nucleic acid including a modification incapable of elongation at the 3'-terminal thereof,
a primer complementary to the sequence in the vicinity of the 3'-terminal of the target nucleic acid, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

3. A method of preparing a detection target structure useful for detecting the target nucleotide sequence, comprising:
Reacting a first target nucleic acid which contains a first target nucleotide sequence positioned in the vicinity of 3'-terminal thereof, and a second target nucleic acid which is complementary to the first target nucleic acid and contains a second target nucleotide sequence positioned in the vicinity of 5'-terminal thereof, under the conditions that permit obtaining an appropriate elongation reaction, with;
a first primer complementary to the sequence of a region of the first target nucleic acid, said region being positioned on the 5'-terminal side relative to the 5'-terminal of the first target nucleotide sequence,
a stopper nucleic acid complementary to the sequence of a region of the second target nucleic acid, said region being positioned on the 3'-terminal side relative to the 3'-terminal of the second target nucleotide sequence, and said stopper nucleic acid including a modification incapable of elongation at the 3'-terminal thereof,
a second primer complementary to the sequence in the vicinity of the 3'-terminal of the second target nucleic acid, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

4. A method of preparing a detection target structure useful for detecting the target nucleotide sequence, comprising:
reacting a target nucleic acid which contains a first target nucleotide sequence positioned in the vicinity of 3'-terminal thereof and a second target nucleotide sequence positioned in the vicinity of 5'-terminal thereof, under the conditions that permit an appropriate elongation reaction, with;
a primer complementary to the sequence of a region of the target nucleic acid, said region being positioned on the 5'-terminal side relative to the 5'-terminal of the first target nucleotide sequence,
a stopper nucleic acid complementary to the sequence of a region of the target nucleic acid, said region being positioned on the 3'-terminal side relative to the 3'-terminal of the second target nucleotide sequence, and said stopper nucleic acid including a modification incapable of elongation at the 3'-terminal thereof, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

5. A method of preparing a detection target structure useful for detecting the target nucleotide sequence, comprising:
reacting a target nucleic acid which contains a first target nucleotide sequence positioned in the vicinity of 3'-terminal thereof, a second target nucleotide sequence positioned on the 5'-terminal side relative to the first target nucleotide sequence, a third target nucleotide sequence positioned on the 5'-terminal side relative to the second target nucleotide sequence, and a fourth target nucleotide sequence positioned in the vicinity of 5'-terminal thereof, under the conditions that permit an appropriate elongation reaction, with;
first, second and third primers complementary to the sequences of regions of the target nucleic acid, each of said regions being positioned on the 5'-terminal side relative to the 5'-terminal of each of first, second and third target nucleotide sequences
first, second and third stopper nucleic acids complementary to the sequences of regions of the target nucleic acid, each of said regions being positioned on the 3'-terminal side relative to the 3'-terminal of each of the second, third and fourth target nucleotide sequences, and said stopper nucleic acids including a modification incapable of elongation on the 3'-terminal thereof, and
an enzyme having a nucleotide elongation activity,
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

6. A method of preparing a detection target structure useful for detecting the target nucleotide sequence, comprising:
(1) reacting a double stranded nucleic acid which comprises a target nucleic acid having a target nucleotide sequence in the vicinity of 3'-terminal thereof and a complementary strand of the target nucleic acid, under the conditions that permit an appropriate amplification reaction, with;
a first primer including at least a sequence which is formed of a nucleic acid not resistant to a nuclease enzyme and complementary to a target nucleotide sequence and another sequence which is formed of a nucleic acid resistant to a nuclease enzyme and complementary to a region of the target nucleic acid, said region being positioned on the 5'-terminal side relative to the 5'-terminal of the target nucleotide sequence,
a second primer which is complementary to the sequence in the vicinity of the 5'-terminal of the target nucleic acid and contains a nucleic acid resistant to a nuclease enzyme at the 5'-terminal, and
an enzyme having a nucleotide elongation activity; and
(2) digesting the amplified product obtained by the reaction in step (1) with a nuclease enzyme capable of decomposing the nucleic acid not resistant to the nuclease enzyme;
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

7. A method of preparing a detection target structure useful for detecting the target nucleotide sequence, comprising:
(1) reacting a double stranded nucleic acid comprising a first target nucleic acid which has a first target nucleotide sequence in the vicinity of 3'-terminal thereof and a second target nucleic acid which is complementary to the first target nucleic acid and has a second target nucleotide sequence in the vicinity of 5'-terminal thereof, under the conditions that permit an appropriate amplification reaction, with;
a first primer including at least a sequence which is formed of a nucleic acid not resistant to a nuclease enzyme and complementary to the first target nucleotide sequence and another sequence which is formed of a nucleic acid resistant to a nuclease enzyme and complementary to a region of the first target nucleic acid, said region being positioned on the 5'-terminal side relative to the 5'-terminal of the first target nucleotide sequence,
a second primer including at least a sequence which is formed of a nucleic acid not resistant to a nuclease enzyme and complementary to the second target nucleotide sequence and another sequence which is formed of a nucleic acid resistant to a nuclease enzyme and complementary to a region of the second target nucleic acid, said region being positioned on the 5'-terminal side relative to the 5'-terminal of the second target nucleotide sequence, and
an enzyme having a nucleotide elongation activity; and
(2) digesting the amplified product obtained by the reaction in step (1) with a nuclease enzyme;
thereby obtaining a detection target structure comprising a single stranded nucleic acid portion and a double stranded nucleic acid portion, the single strand nucleic acid portion comprising the target nucleotide sequence, and the double strand nucleic acid portion comprising residual portion of the target nucleic acid excluding at least the target nucleotide sequence and a protective strand complementary to the residual portion of the target nucleic acid.

8. A method of detecting a target nucleotide sequence, comprising:
(1) preparing a detection target structure on the basis of the target nucleic acid contained in a sample by employing the method defined in any one of claims 1 to 7;
(2) allowing a nucleic acid probe complementary to the target nucleotide sequence to react with the detection target structure prepared in step (1) under the conditions that permit an appropriate hybridization; and
(3) detecting the hybridization occurred by the reaction in step (2) so as to determine the presence of the target nucleotide sequence.

9. A detection target structure obtained by the method defined in any of claims 1 to 7.

10. An assay kit for detecting a target nucleotide sequence by performing the detection method defined in claim 8, comprising at least one constituting element selected from the group consisting of a primer, an enzyme having a nucleotide elongation activity, a stopper nucleic acid, an enzyme for amplifying a nucleic acid, a nuclease enzyme, and the detection target structure defined in claim 9.
